(19) Europäisches Patentamt
European Patent Office
Office européen des brevets

(11) EP 0 345 671 B1

(12) **EUROPEAN PATENT SPECIFICATION**

(45) Date of publication and mention of the grant of the patent: **21.08.1996 Bulletin 1996/34**

(51) Int. Cl.$^6$: **C07D 501/46**, A61K 31/545, C07D 501/18

(21) Application number: **89110068.7**

(22) Date of filing: **03.06.1989**

# (54) Cephem compounds and processes for preparation thereof

Cephemverbindungen und Verfahren zu ihrer Herstellung

Composés céphem et procédés pour leur préparation

(84) Designated Contracting States:
**AT BE CH DE ES FR GB GR IT LI LU NL SE**

(30) Priority: **06.06.1988 GB 8813308**
**15.06.1988 GB 8814196**
**01.11.1988 GB 8825518**

(43) Date of publication of application:
**13.12.1989 Bulletin 1989/50**

(73) Proprietor: **FUJISAWA PHARMACEUTICAL CO., LTD.**
**Osaka-shi Osaka 541 (JP)**

(72) Inventors:
- **Yamanaka, Hideaki**
  **Hirakata-shi Osaka 573 (JP)**
- **Yoshida, Yoshiki**
  **Suita-shi Osaka 565 (JP)**
- **Goto, Jiro**
  **Suita-shi Osaka 565 (JP)**
- **Terasawa, Takeshi**
  **Ikeda-shi Osaka 563 (JP)**
- **Okuda, Shinya**
  **Ikeda-shi Osaka 563 (JP)**
- **Sakane, Kazuo**
  **Kawanishi-shi Hyogo 666-01 (JP)**

(74) Representative: **Türk, Gille, Hrabal, Leifert**
**Brucknerstrasse 20**
**40593 Düsseldorf (DE)**

(56) References cited:
**EP-A- 0 192 176** **EP-A- 0 267 733**

- **CHEMICAL ABSTRACTS, vol. 107, no. 13, 28th September 1987, page 612, column 2, abstract no. 115433p, Columbus, Ohio, US; & JP-A- 62 30 788 (BANYU PHARMACEUTICAL CO., LTD) 09-02-1987**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 345 671 B1

## Description

The present invention relates to new cephem compounds and pharmaceutically acceptable salts thereof. More particularly, it relates to new cephem compounds and pharmaceutically acceptable salts thereof, which have antimicrobial activities, to processes for preparation thereof, and to pharmaceutical composition comprising the same.

Accordingly, one object of the present invention is to provide the cephem compounds and pharmaceutically acceptable salts thereof, which are highly active against a number of pathogenic microorganisms.

Another object of the present invention is to provide processes for the preparation of the cephem compounds and salts thereof.

A further object of the present invention is to provide pharmaceutical composition comprising, as an active ingredient, said cephem compounds or their pharmaceutically acceptable salts.

EP-A-0 192 176 discloses cephem compounds of the formula

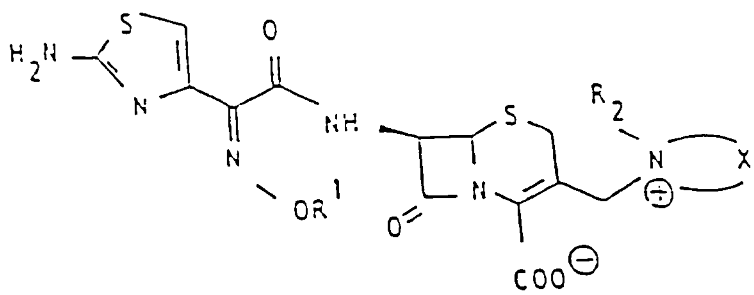

and the use of such cephem compounds as a medicament, especially for the treamtment of infectious diseases.

EP-A-0 267 733 discloses cephalosporine compounds having a 3-position substituent of the formula

$$CH_2\text{-}Y\text{-}Q$$

wherein Y is a linking group of a certain constitution and Q a benzene ring which may be substituted or Q is a group of the formula

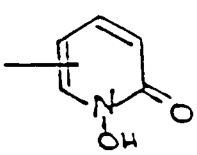

or

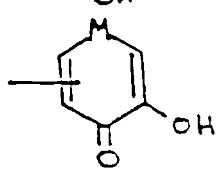

Chemical abstracts, Vol. 107, no. 13, page 612, column 2, abstract no. 115433 p corresponding to JP-A-6230788 discloses cephalosporine derivates of the formula:

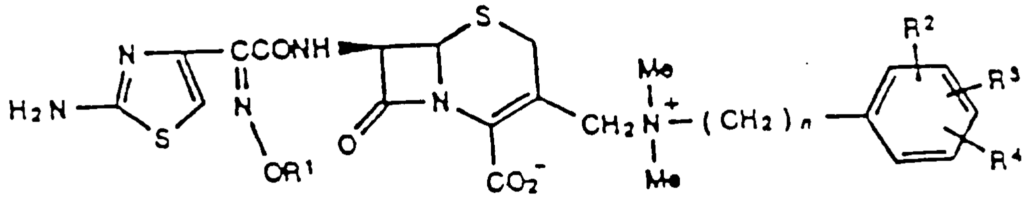

The object cephem compounds are novel and can be represented by the following general formula (I) :

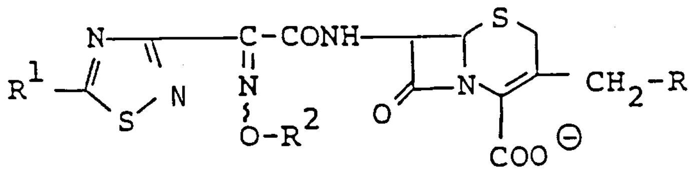

(I)

wherein

| | |
|---|---|
| $R^1$ | is amino or a protected amino group, |
| $R^2$ | is $C_1$-$C_6$alkyl, $C_2$-$C_6$alkenyl, carboxy($C_1$-$C_6$)alkyl or protected carboxy($C_1$-$C_6$)alkyl, and |
| R | is a group of the formula : |

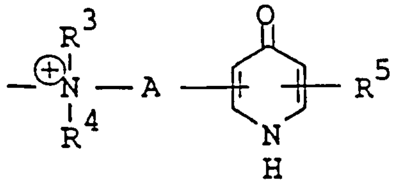

| | |
|---|---|
| | (in which |
| $R^3$ and $R^4$ | are each $C_1$-$C_6$ alkyl, or |
| $R^3$ and $R^4$ | are linked together to form $C_3$-$C_6$ alkylene, |
| A | is ($C_1$-$C_6$) alkylene, and |
| $R^5$ | is hydroxy or a protected hydroxy group). |

The cephem compound (I) of the present invention can be prepared by processes as illustrated in the following.

Process 1

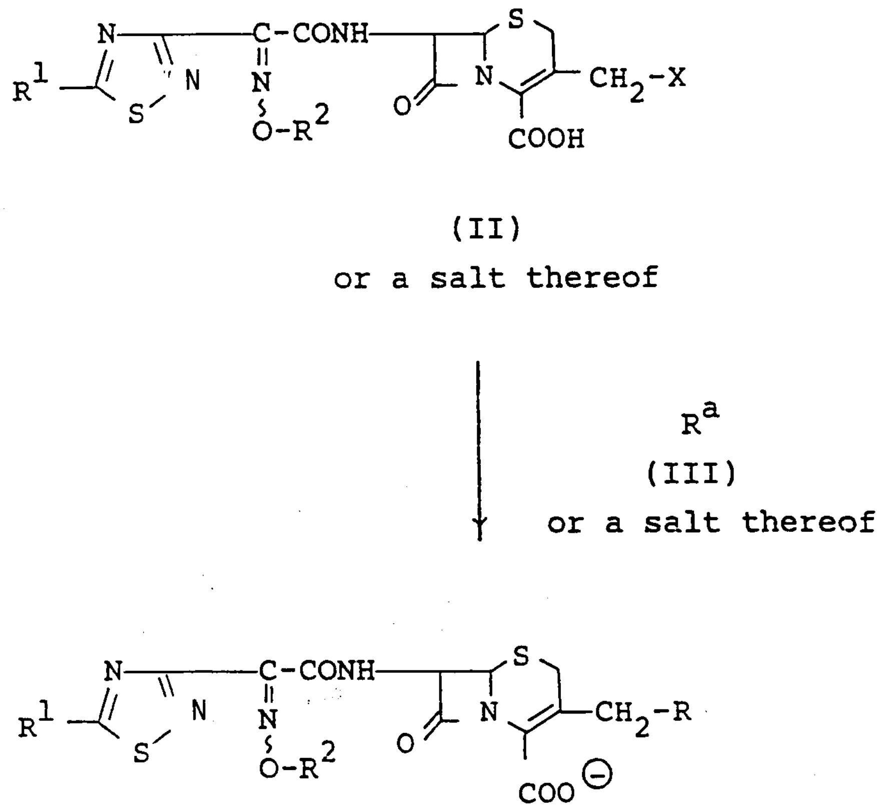


(II)
or a salt thereof

(I)
or a salt thereof

Process 2

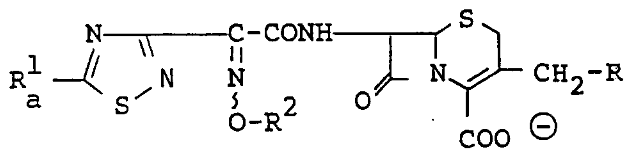

(Ia)

or a salt thereof

Elimination reaction of the amino protective group

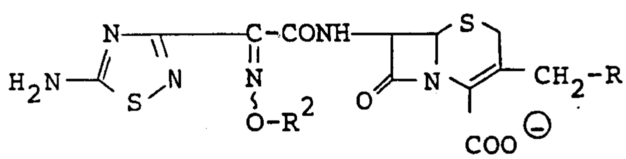

(Ib)

or a salt thereof

Process 3

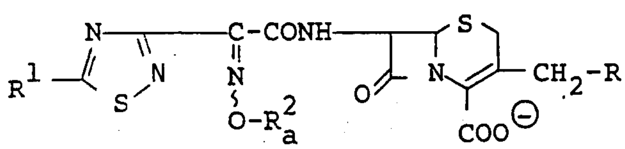

(Ic)

or a salt thereof

Elimination reaction of the carboxy protective group

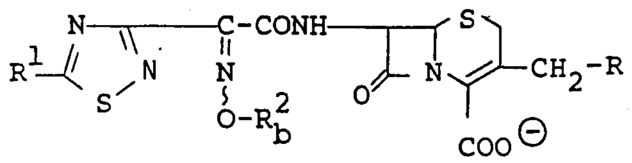

(Id)

or a salt thereof

Process 4

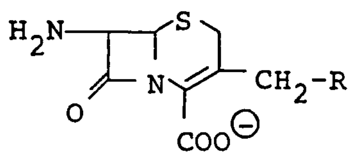

(IVb)

or its reactive derivative at the amino group or a salt thereof

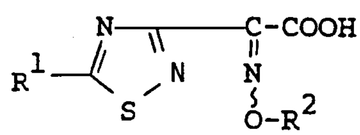

(V)

or its reactive derivative at the carboxy group or a salt thereof

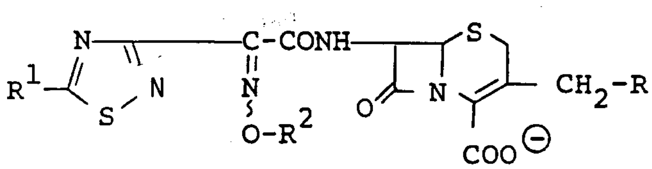

(I)

wherein

| | |
|---|---|
| R, $R^1$ and $R^2$ | are each as defined above, |
| X | is an acid residue, |

$R^a$ is a compound of the formula :

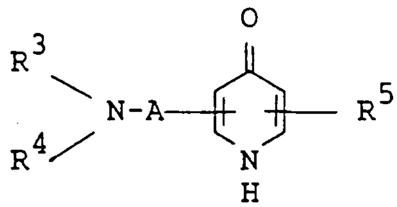

(in which $R^3$, $R^4$, $R^5$ and A are each as defined above),

$R_a^1$ is a protected amino group,

$R_a^2$ is protected carboxy $(C_1-C_6)$,alkyl, and

$R_b^2$ is carboxy$(C_1-C_6)$ alkyl.

Some of the starting compounds are novel and can be prepared by processes as illustrated in the following.

Process A

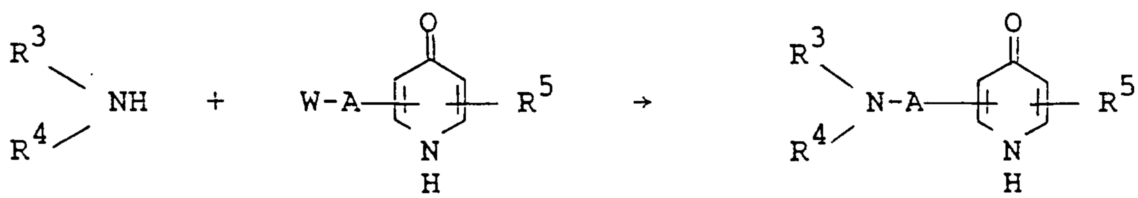

(VIII) or a salt thereof

(IX) or a salt thereof

(IIIa) or a salt thereof

Process B

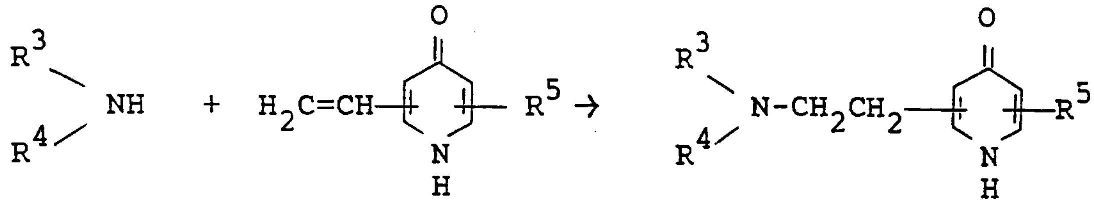

(VIII) or a salt thereof

(X) or a salt thereof

(IIIb) or a salt thereof

Process C

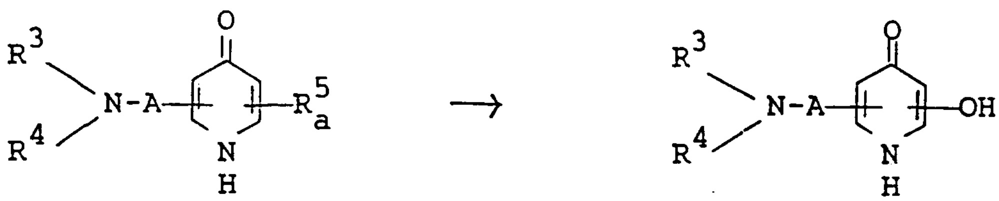

(IIIc) or a salt thereof

(IIId) or a salt thereof

Process D

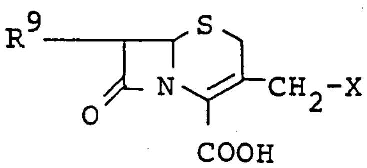

+ $R^a$

(XI) or a salt thereof

(III) or a salt thereof

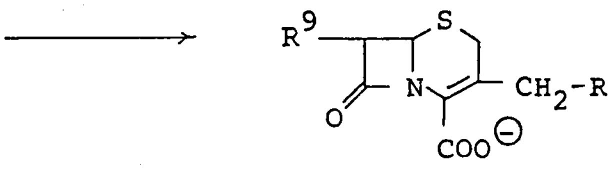

(IV)
or a salt thereof

Process E

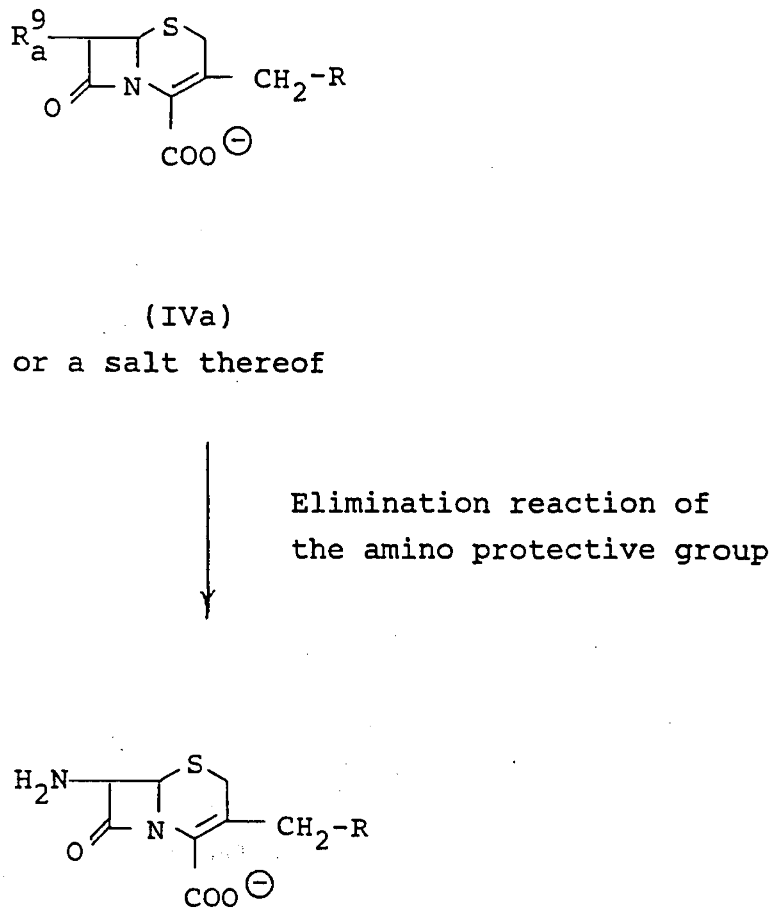


(IVa)
or a salt thereof

(IVb)
or a salt thereof

wherein

$R^3$, $R^4$, $R^5$, A, X, R and $R^a$ are each as defined above,

W is an acid residue,

| | |
|---|---|
| $R^5_a$ | is a protected hydroxy group, |
| $R^9$ | is amino or a protected amino group, and |
| $R^9_a$ | is a protected amino group. |

Regarding the compounds (I), (Ia)~(Id), (II) and (V), it is to be understood that said compounds include syn isomer, anti isomer and a mixture thereof.

For example, with regard to the object compounds (I), syn isomer means one geometrical isomer having the partial structure represented by the following formula :

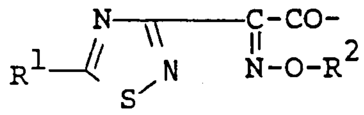

(wherein $R^1$ and $R^2$ are each as defined above) and anti isomer means the other geometrical isomer having the partial structure represented by the following formula :

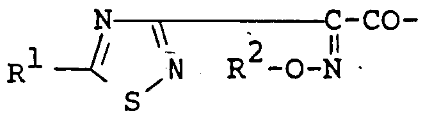

(wherein $R^1$ and $R^2$ are each as defined above), and all of such geometrical isomers and mixture thereof are included within the scope of this invention.

In the present specification and claim, the partial structure of these geometrical isomers and mixture thereof is represented for convenience sake by the following formula :

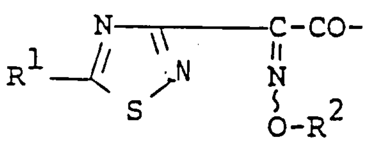

(wherein $R^1$ and $R^2$ are each as defined above).

Further, regarding the compounds (I), (Ia)~(Id), (III), (IIIa), (IIIb), (IIIc), (IIId), (IV), (IVa), (IVb), (IX) and (X), it is to be understood that the said compounds include tautomeric isomers. For example, with regard to the object compound (I), in case that the symbol "R" in the compound (I) means the group represented by the following formula :

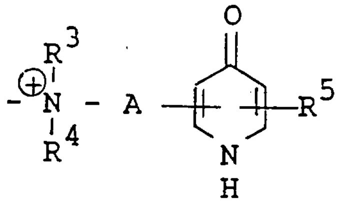

(wherein $R^3$, $R^4$, $R^5$ and A are each as defined above), said group can also exist in the tautomeric form and such tautomeric equilibrium can be represented by the following scheme.

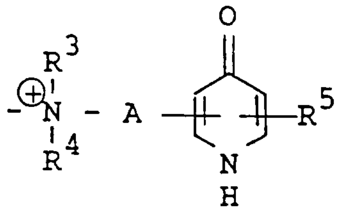

(A')

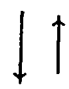

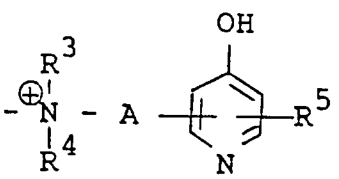

(B')

Both of the above tautomeric isomers are included within the scope of the present invention. In the present specification and claim, the compounds including the group of such tautomeric isomers are represented for the convenient sake by one expression of the group of the formula (A').

In the above and subsequent descriptions of the present specification, suitable examples and illustration of the various definitions which the present invention include within the scope thereof are explained in detail as follows.

Suitable "protected amino" may include an acylamino or an amino group substituted by a conventional protecting group such as ar($C_1$-$C_6$)alkyl which may have suitable substituent(s) (e.g. benzyl, trityl, etc.) or the like.

Suitable "acyl moiety" in the term "acylamino" may include carbamoyl, aliphatic acyl group and acyl group containing an aromatic or heterocyclic ring. And, suitable examples of the said acyl may be $C_1$-$C_6$ alkanoyl (e.g. formyl, acetyl, propionyl, butyryl, isobutyryl, valeryl, isovaleryl, oxalyl, succinyl, pivaloyl, etc.); $C_1$-$C_6$ alkoxycarbonyl (e.g. methoxycarbonyl, ethoxycarbonyl, propoxycarbonyl, 1-cyclopropylethoxycarbonyl, isopropoxycarbonyl, butoxycarbonyl, tertiarybutoxycarbonyl, pentyloxycarbonyl, hexyloxycarbonyl, etc.);
$C_1$-$C_6$ alkanesulfonyl (e.g. mesyl, ethanesulfonyl, propanesulfonyl, isopropanesulfonyl, butanesulfonyl, etc.); arenesulfonyl (e.g. benzenesulfonyl, tosyl, etc.); aroyl (e.g. benzoyl, toluoyl, xyloyl, naphthoyl, phthaloyl, indancarbonyl, etc.); ar($C_1$-$C_6$)alkanoyl (e.g. phenylacetyl, phenylpropionyl, etc.);
ar($C_1$-$C_6$)alkoxycarbonyl (e.g. benzyloxycarbonyl, phenethyloxycarbonyl, etc.), and the like. The acyl moiety as stated above may have suitable substituent(s) such as halogen (e.g. chlorine, bromine, iodine or fluorine) or the like.

Suitable "organic group" may include $C_1$-$C_6$ alkyl (e.g., methyl, ethyl, propyl, isopropyl, butyl, isobutyl, tert-butyl, pentyl, neopentyl, tert-pentyl, hexyl, etc.),

mono(or di or tri)halo($C_1$-$C_6$)alkyl (e.g. chloromethyl, dichloromethyl, trichloromethyl, bromomethyl, chloroethyl, dichloroethyl, trichloroethyl, fluoroethyl, trifluoroethyl, etc.),

$C_2$-$C_6$ alkenyl (e.g., vinyl, 1-propenyl, allyl, 1-methylallyl, 1 or 2 or 3-butenyl, 1 or 2 or 3 or 4-pentenyl, 1 or 2 or 3 or 4 or 5-hexenyl, etc.),

$C_2$-$C_6$ alkynyl (e.g., ethynyl, 1-propynyl, propargyl, 1-methylpropargyl, 1 or 2 or 3 butynyl, 1 or 2 or 3 or 4-pentynyl, 1 or 2 or 3 or 4 or 5-hexynyl, etc.),

aryl (e.g., phenyl,naphthyl, etc.),

ar($C_1$-$C_6$)alkyl such as phenyl($C_1$-$C_6$)alkyl (e.g., benzyl, phenethyl, phenylpropyl, etc.),

carboxy($C_1$-$C_6$)alkyl wherein $C_1$-$C_6$ alkyl moiety can be referred to the ones as exemplified above, protected car-

boxy($C_1$-$C_6$)alkyl wherein $C_1$-$C_6$ alkyl moiety can be referred to the ones as exemplified above and protected carboxy moiety can be referred to the ones as exemplified below, and the like.

Suitable "protected carboxy moiety" in the term "protected carboxy($C_1$-$C_6$)alkyl" may include esterified carboxy and the like. And suitable examples of said ester may be the ones such as $C_1$-$C_6$ alkyl ester (e.g., methyl ester, ethyl ester, propyl ester, isopropyl ester, butyl ester, isobutyl ester, t-butyl ester, pentyl ester, t-pentyl ester, hexyl ester, 1-cyclopropylethyl ester, etc.);
$C_2$-$C_6$ alkenyl ester (e.g., vinyl ester, allyl ester, etc.);
$C_2$-$C_6$ alkynyl ester (e.g., ethynyl ester, propynyl ester, etc.);
$C_1$-$C_6$ alkoxyalkyl ester (e.g., methoxymethyl ester, ethoxymethyl ester, isopropoxymethyl ester, 1-methoxyethyl ester, 1-ethoxyethyl ester, etc.);
$C_1$-$C_6$ alkylthioalkyl ester (e.g., methylthiomethyl ester, ethylthiomethyl ester, ethylthioethyl ester, isopropylthiomethyl ester, etc.);
mono(or di or tri)halo($C_1$-$C_6$)alkyl ester (e.g. 2-iodoethyl ester, 2,2,2-trichloroethyl ester, etc.); $C_1$-$C_6$ alkanoyloxy($C_1$-$C_6$)alkyl ester (e.g., acetoxymethyl ester, propionyloxymethyl ester, butyryloxymethyl ester, valeryloxymethyl ester, pivaloyloxymethyl ester, hexanoyloxymethyl ester, 2-acetoxyethyl ester, 2-propionyloxyethyl ester, etc.);
$C_1$-$C_6$ alkanesulfonyl($C_1$-$C_6$)alkyl ester (e.g. mesylmethyl ester, 2-mesylethyl ester etc.);
ar($C_1$-$C_6$)alkyl ester, for example, phenyl($C_1$-$C_6$)alkyl ester which may have one or more suitable substituent(s) (e.g., benzyl ester, 4-methoxybenzyl ester, 4-nitrobenzyl ester, phenethyl ester, trityl ester, benzhydryl ester, bis(methoxyphenyl)methyl ester, 3,4-dimethoxybenzyl ester, 4-hydroxy-3,5-di-t-butylbenzyl ester, etc.);
aryl ester which may have one or more suitable substituent(s) such as substituted or unsubstituted phenyl ester (e.g., phenyl ester, tolyl ester, t-butylphenyl ester, xylyl ester, mesityl ester, cumenyl ester, 4-chlorophenyl ester, 4-methoxyphenyl ester, etc.);
tri($C_1$-$C_6$)alkyl silyl ester;
$C_1$-$C_6$ alkylthioester (e.g. methylthioester, ethylthioester, etc.) and the like.

Suitable "$C_1$-$C_6$ alkyl" can be referred to the ones as exemplified above.

Suitable "$C_3$-$C_6$ alkylene" may include trimethylene, tetramethylene, pentamethylene and hexamethylene.

Suitable "$C_1$-$C_6$ alkylene" may include methylene, ethylene, trimethylene, tetramethylene, pentamethylene, hexamethylene and the like.

Suitable "protective group" in the "protected hydroxy group" may include acyl as mentioned above, tetrahydropyranyl and the like.

Suitable "acid residue" may include halogen [e.g. chlorine, bromine, iodine, etc.], acyloxy such as sulfonyloxy [e.g. benzenesulfonyloxy, tosyloxy, mesyloxy, etc.], $C_2$-$C_6$ alkanoyloxy [e.g. acetyloxy, propionyloxy, etc.] or the like.

Suitable pharmaceutically acceptable salts of the object compound (I) are conventional non-toxic salts and include a metal salt such as an alkali metal salt [e.g. sodium salt, potassium salt, etc.] and an alkaline earth metal salt [e.g. calcium salt, magnesium salt, etc.], an ammonium salt, an organic base salt [e.g. trimethylamine salt, triethylamine salt, pyridine salt, picoline salt, dicyclohexylamine salt, N,N'-dibenzylethylenediamine salt, etc], an organic acid salt [e.g. formate, acetate, trifluoroacetate, maleate, tartrate, methanesulfonate, benzenesulfonate, toluenesulfonate, etc.], an inorganic acid salt [e.g. hydrochloride, hydrobromide, sulfate, phosphate, etc.], a salt with an amino acid [e.g. arginine salt, aspartic acid salt, glutamic acid salt, etc.], and the like.

Preferred embodiments of the object compound (I) are as follows.

| | |
|---|---|
| $R^1$ | is amino or acylamino (more preferably $C_1$-$C_6$ alkanoylamino), |
| $R^2$ | is $C_1$-$C_6$ alkyl, $C_2$-$C_6$ alkenyl, carboxy($C_1$-$C_6$)alkyl or protected carboxy($C_1$-$C_6$)alkyl [more preferably esterified carboxy($C_1$-$C_6$)alkyl, most preferably $C_1$-$C_6$ alkoxycarbonyl($C_1$-$C_6$)alkyl], |
| $R^3$ and $R^4$ | are each $C_1$-$C_6$ alkyl, or |
| $R^3$ and $R^4$ | are linked together to form $C_3$-$C_6$ alkylene, |
| A | is $C_1$-$C_6$ alkylene (more preferably $C_1$-$C_3$ alkylene), and |
| $R^5$ | is hydroxy. |

The processes for preparing the object and starting compounds of the present invention are explained in detail in the following.

Process 1

The compound (I) or a salt thereof can be prepared by reacting the compound (II) or a salt thereof with the compound (III) or a salt thereof.

Suitable salts of the compounds (II) and (III) can be referred to the ones as exemplified for the compound (I).

The present reaction may be carried out in a solvent such as water, phosphate buffer, acetone, chloroform, acetonitrile, methylene chloride, ethylene chloride, formamide, N,N-dimethylformamide, methanol, ethanol, diethyl ether, tetrahydrofuran, dimethyl sulfoxide, or any other organic solvent which does not adversely affect the reaction. Among the solvents, hydrophilic solvents may be used in a mixture with water. The reaction temperature is not critical, and the reaction is usually carried out under cooling, at ambient temperature or under warming.

Process 2

The compound (Ib) or a salt thereof can be prepared by subjecting the compound (Ia) or a salt thereof to elimination reaction of the amino protective group.

Suitable salts of the compounds (Ia) and (Ib) can be referred to the ones as exemplified for the compound (I).

This reaction is carried out in accordance with a conventional method such as hydrolysis, reduction or the like.

The hydrolysis is preferably carried out in the presence of a base or an acid including Lewis acid. Suitable base may include an inorganic base and an organic base such as an alkali metal [e.g. sodium, potassium, etc.], an alkaline earth metal [e.g. magnesium, calcium, etc.], the hydroxide or carbonate or bicarbonate thereof, trialkylamine [e.g. trimethylamine, triethylamine, etc.], picoline, 1,5-diazabicyclo[4.3.0]non-5-ene, 1,4-diazabicyclo[2.2.2]octane, 1,8-diazabicyclo[5.4.0]-undec-7-ene, or the like.

Suitable acid may include an organic acid [e.g. formic acid, acetic acid, propionic acid, trichloroacetic acid, trifluoroacetic acid, etc.] and an inorganic acid [e.g. hydrochloric acid, hydrobromic acid, sulfuric acid, hydrogen chloride, hydrogen bromide, etc.]. The elimination using Lewis acid such as trihaloacetic acid [e.g. trichloroacetic acid, trifluoroacetic acid, etc.] or the like is preferably carried out in the presence of cation trapping agents [e.g. anisole, phenol, etc.].

The reaction is usually carried out in a solvent such as water, an alcohol [e.g. methanol, ethanol, etc.], methylene chloride, tetrahydrofuran, a mixture thereof or any other solvent which does not adversely affect the reaction. A liquid base or acid can be also used as the solvent. The reaction temperature is not critical and the reaction is usually carried out under cooling to warming.

The reduction method applicable for the elimination reaction may include chemical reduction and catalytic reduction.

Suitable reducing agents to be used in chemical reduction are a combination of metal [e.g. tin, zinc, iron, etc.] or metallic compound [e.g. chromium chloride, chromium acetate, etc.] and an organic or inorganic acid [e.g. formic acid, acetic acid, propionic acid, trifluoroacetic acid, p-toluenesulfonic acid, hydrochloric acid, hydrobromic acid, etc.].

Suitable catalysts to be used in catalytic reduction are conventional ones such as platinum catalysts [e.g. platinum plate, spongy platinum, platinum black, colloidal platinum, platinum oxide, platinum wire, etc.], palladium catalysts [e.g. spongy palladium, palladium black, palladium oxide, palladium on carbon, colloidal palladium, palladium on barium sulfate, palladium on barium carbonate, etc.], nickel catalysts [e.g. reduced nickel, nickel oxide, Raney nickel, etc.], cobalt catalysts [e.g. reduced cobalt, Raney cobalt, etc.], iron catalysts [e.g. reduced iron, Raney iron, etc.], copper catalysts [e.g. reduced copper, Raney copper, Ullman copper, etc.] and the like.

The reduction is usually carried out in a conventional solvent which does not adversely affect the reaction such as water, methanol, ethanol, propanol, N,N-dimethylformamide, or a mixture thereof. Additionally, in case that the above-mentioned acids to be used in chemical reduction are in liquid, they can also be used as a solvent, further, a suitable solvent to be used in catalytic reduction may be the above-mentioned solvent, and other conventional solvent such as diethyl ether, dioxane, tetrahydrofuran, alcohol (e.g. methanol, ethanol, etc.), etc., or a mixture thereof.

The reaction temperature of this reduction is not critical and the reaction is usually carried out under cooling to warming.

Process 3

The compound (Id) or a salt thereof can be prepared by subjecting the compound (Ic) or a salt thereof to elimination reaction of the carboxy protective group.

Suitable salts of the compound (Ic) and (Id) can be referred to the ones as exemplified for the compound (I).

This elimination can be carried out in a similar manner to that of the aforementioned Process 2, and therefore the reagents to be used and the reaction conditions (e.g. solvent, reaction temperature, etc.) can be referred to those of the Process 2.

Process 4

The compound (I) or a salt thereof can be prepared by reacting the compound (IVb) or its reactive derivative at the amino group or a salt thereof with the compound (V) or its reactive derivative at the carboxy group or a salt thereof.

Suitable reactive derivative at the amino group of the compound (IVb) may include Schiff's base type imino or its tautomeric enamine type isomer formed by the reaction of the compound (IVb) with a carbonyl compound such as aldehyde, ketone or the like; a silyl derivative formed by the reaction of the compound (IVb) with a silyl compound such as bis(trimethylsilyl)acetamide, mono(trimethylsilyl)acetamide [e.g. N-(trimethylsilyl)acetamide], bis(trimethylsilyl)urea or the like; a derivative formed by reaction of the compound (IVb) with phosphorus trichloride or phosgene, and the like.

Suitable salts of the compound (IVb) and its reactive derivative can be referred to the ones as exemplified for the compound (I).

Suitable reactive derivative at the carboxy group of the compound (V) may include an acid halide, an acid anhydride, an activated amide, an activated ester, and the like. Suitable examples of the reactive derivatives may be an acid chloride; an acid azide; a mixed acid anhydride with an acid such as substituted phosphoric acid [e.g. dialkylphosphoric acid, phenylphosphoric acid, diphenylphosphoric acid, dibenzylphosphoric acid, halogenated phosphoric acid, etc.], dialkylphosphorous acid, sulfurous acid, thiosulfuric acid, sulfuric acid, sulfonic acid [e.g. methanesulfonic acid, etc.], aliphatic carboxylic acid [e.g. acetic acid, propionic acid, butyric acid, isobutyric acid, pivalic acid, pentanoic acid, isopentanoic acid, 2-ethylbutyric acid, trichloroacetic acid, etc.] or aromatic carboxylic acid [e.g. benzoic acid, etc.]; a symmetrical acid anhydride; an activated amide with imidazole, 4-substituted imidazole, dimethylpyrazole, triazole or tetrazole; or an activated ester [e.g. cyanomethyl ester, methoxymethyl ester, dimethyliminomethyl [$(CH_3)_2\overset{+}{N}=CH-$] ester, vinyl ester, propargyl ester, p-nitrophenyl ester, 2,4-dinitrophenyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester, etc.], or an ester with a N-hydroxy compound [e.g. N,N-dimethylhydroxylamine, 1-hydroxy-2-(1H)-pyridone, N-hydroxysuccinimide, N-hydroxyphthalimide, 1-hydroxy-1H-benzotriazole, etc.], and the like. These reactive derivatives can optionally be selected from them according to the kind of the compound (V) to be used.

Suitable salts of the compound (V) and its reactive derivative can be referred to the ones as exemplified for the compound (I).

The reaction is usually carried out in a conventional solvent such as water, alcohol [e.g. methanol, ethanol, etc.], acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. These conventional solvent may also be used in a mixture with water.

In this reaction, when the compound (V) is used in a free acid form or its salt form, the reaction is preferably carried out in the presence of a conventional condensing agent such as N,N'-dicyclohexylcarbodiimide; N-cyclohexyl-N'-morpholinoethylcarbodiimide; N-cyclohexyl-N'-(4-diethylaminocyclohexyl)carbodiimide; N,N'-diethylcarbodiimide, N,N'-diisopropylcarbodiimide; N-ethyl-N'-(3-dimethylaminopropyl)carbodiimide; N,N'-carbonylbis-(2-methylimidazole); pentamethyleneketene-N-cyclohexylimine; diphenylketene-N-cyclohexylimine; ethoxyacetylene; 1-alkoxy-1-chloroethylene; trialkyl phosphite; ethyl polyphosphate; isopropyl polyphosphate; phosphorus oxychloride (phosphoryl chloride); phosphorus trichloride; thionyl chloride; oxalyl chloride; $C_1$-$C_6$ alkyl haloformate [e.g. ethyl chloroformate, isopropyl chloroformate, etc.]; triphenylphosphine; 2-ethyl-7-hydroxybenzisoxazolium salt; 2-ethyl-5-(m-sulfophenyl)isoxazolium hydroxide intramolecular salt; 1-(p-chlorobenzenesulfonyloxy)-6-chloro-1H-benzotriazole; so-called Vilsmeier reagent prepared by the reaction of N,N-dimethylformamide with thionyl chloride, phosgene, trichloromethyl chloroformate, phosphorus oxychloride, etc., or the like.

The reaction may also be carried out in the presence of an inorganic or organic base such as an alkali metal bicarbonate, tri($C_1$-$C_6$)alkylamine, pyridine, N-($C_1$-$C_6$)alkylmorpholine, N,N-di($C_1$-$C_6$)alkylbenzylamine, or the like.

The reaction temperature is not critical, and the reaction is usually carried out under cooling to warming.

<u>Process A</u>

The compound (IIIa) or a salt thereof can be prepared by reacting the-compound (VIII) or a salt thereof with the compound (IX) or a salt thereof.

The present reaction is usually carried out in the presence of a base.

Suitable base can be referred to the ones as exemplified in <u>Process 2</u>.

The reaction is usually carried out in a solvent such as water, an alcohol [e.g. methanol, ethanol, etc.], methylene chloride, tetrahydrofuran, a mixture thereof or any other solvent which does not adversely affect the reaction. The reaction temperature is not critical and the reaction is usually carried out under cooling to warming.

<u>Process B</u>

The compound (IIIb) or a salt thereof can be prepared by reacting the compound (VIII) or a salt thereof with the compound (X) or a salt thereof.

The reaction is usually carried out in the presence or absence of a solvent such as water, an alcohol [e.g. methanol, ethanol, etc.], methylene chloride, tetrahydrofuran, a mixture thereof or any other solvent which does not adversely affect the reaction. The reaction temperature is not critical and the reaction is usually carried out under warming to heating.

Process C

The compound (IIId) or a salt thereof can be prepared by subjecting the compound (IIIc) or a salt thereof to elimination reaction of the hydroxy protective group.

The elimination reaction can be carried out in a similar manner to that of the aforementioned Process 2, and therefore the reagents to be used and the reaction conditions (e.g. solvent, reaction temperature, etc.) can be referred to those of the Process 2.

Process D

The compound (IV) or a salt thereof can be prepared by reacting the compound (XI) or a salt thereof with the compound (III) or a salt thereof.

This reaction can be carried out in a similar manner to that of the aforementioned Process 1, and therefore the reagents to be used and the reaction conditions (e.g. solvent, reaction temperature, etc.) can be referred to those of the Process 1.

Process E

The compound (IVb) or a salt thereof can be prepared by subjecting the compound (IVa) or a salt thereof to elimination reaction of the amino protective group.

This elimination can be carried out in a similar manner to that of the aforementioned Process 2, and therefore the reagents to be used and the reaction conditions (e.g. solvent, reaction temperature, etc.) can be referred to those of the Process 2.

The object compound (I) and pharmaceutically acceptable salts thereof are novel and exhibit high antimicrobial activity, inhibiting the growth of a wide variety of pathogenic microorganisms including Gram-positive and Gram-negative microorganisms and are useful as antimicrobial agents.

Now in order to show the utility of the object compound (I), the test data on MIC (minimal inhibitory concentration) of representative compound of this invention are shown in the following.

Test method :

In vitro antibacterial activity was determined by the two-fold agar-plate dilution method as described below.

One loopful of an overnight culture of each test strain in Trypticase-soy broth ($10^8$ viable cells per ml) was streaked on heart infusion agar (HI-agar)containing graded concentrations of representative test compound, and the minimal inhibitory concentration (MIC) was expressed in terms of µg/mℓ after incubation at 37°C for 20 hours.

Test compound :

(1) 7β-[2-(5-Amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer)

Test result

MIC (µg/ml)

| Test compounds / Test strain | (1) |
|---|---|
| P. aeruginosa 26 | ≤ 0.025 |

For therapeutic administration, the object compound (I) and pharmaceutically acceptable salts thereof of the present

invention are used in the form of conventional pharmaceutical preparation which contains said compound as an active ingredient, in admixture with pharmaceutically acceptable carriers such as an organic or inorganic solid or liquid excipient which is suitable for oral, parenteral and external administration.

The pharmaceutical preparations may be in solid form such as tablet, granule, powder, capsule, or liquid form such as solution, suspension, syrup, emulsion, lemonade and the like.

If needed, there may be included in the above preparations, auxiliary substances, stabilizing agents, wetting agents and other commonly used additives such as lactose, citric acid, tartaric acid, stearic acid, magnesium stearate, terra alba, sucrose, corn starch, talc, gelatin, agar, pectin, peanut oil, olive oil, cacao butter, ethylene glycol, and the like.

While the dosage of the compound (I) may vary from and also depend upon the age, conditions of the patient, a kind of diseases, a kind of the compound (I) to be applied, etc., in general, amounts between 1 mg and about 4,000 mg or even more per day may be administered to a patient. An average single dose of about 50 mg, 100 mg, 250 mg, 500 mg, 1000 mg of the object compound (I) of the present invention may be used in treating diseases infected by pathogenic microorganisms.

The following Preparations and Examples are given for the purpose of illustrating the present invention in more detail.

Preparation 1

To a mixture of 2-chloromethyl-5-benzyloxy-4-pyridone (1.0 g), tetrahydrofuran (10 ml) and water (10 ml) were added dimethylamine hydrochloride (1.31 g) and sodium hydroxide (0.64 g). After being stirred for 1.5 hours, the mixture was concentrated under reduced pressure to dryness. The residue was dissolved in methanol and the insoluble material was filtered off. The filtrate was evaporated in vacuo to give 5-benzyloxy-2-(N,N-dimethylamino)methyl-4-pyridone (1.0 g) as a powder.

IR (Nujol) : 1620 $cm^{-1}$
NMR (DMSO-$d_6$, δ) : 2.20 (6H, s), 3.36 (2H, s), 5.06 (2H, s), 6.28 (1H, s), 7.1 (5H, m), 7.13 (1H, s)

Preparation 2

A solution of 5-benzyloxy-2-chloromethyl-4-pyridone (10.0 g) and triphenylphosphine (10.5 g) in N,N-dimethylformamide (50 ml) was stirred for 5 hours at 90-100°C. The resulting-mixture was poured into ethyl acetate (800 ml). The precipitate was collected by filtration, washed with ethyl acetate and dissolved in dichloromethane (500 ml). To the solution were added water (300 ml) and 38% aqueous formaldehyde (100 ml). The mixture was adjusted to pH 10-10.5 with potassium carbonate. After being stirred for 3 hours at 35-40°C, the organic layer was separated, washed with water, dried over magnesium sulfate and concentrated under reduced pressure. The product was isolated by column chromatography on silica gel with ethyl acetate as an eluent to give 5-benzyloxy-2-vinyl-4-pyridone (6.02 g).

IR (Nujol) : 1640, 1615 $cm^{-1}$
NMR (DMSO-$d_6$, δ) : 5.10 (2H, s), 5.46 (1H, d, J=10Hz), 5.95 (1H, d, J=18Hz), 6.57 (1H, s), 6.63 (1H, dd, J=10Hz, 18Hz), 7.43 (5H, m), 7.66 (1H, s)

Preparation 3

A mixture of 5-benzyloxy-2-vinyl-4-pyridone (2.90 g) and pyrrolidine (5.33 ml) was heated and refluxed for an hour. The mixture was cooled and diluted with tetrahydrofuran (20 ml) and diisopropyl ether (80 ml). After being stirred for an hour at ambient temperature, the resulting precipitate was collected by filtration, washed with diisopropyl ether and air-dried at ambient temperature to give 5-benzyloxy-2-[2-(1-pyrrolidinyl)ethyl]-4-pyridone (3.79 g).

IR (Nujol) : 1633, 1618 $cm^{-1}$
NMR (DMSO-$d_6$, δ) : 1.70 (4H, m), 2.3-2.7 (8H, m), 5.03 (2H, s), 6.15 (1H, s), 7.40 (5H, m), 7.41 (1H, s)

Preparation 4

A mixture of 5-benzyloxy-2-vinyl-4-pyridone (3.12 g), 50% aqueous solution of dimethylamine (15 ml) and ethanol (35 ml) was heated at 100°C in a sealed tube for 8 hours. The resulting mixture was cooled and concentrated under reduced pressure to dryness. The residue was triturated with a mixture of ethyl acetate and diisopropyl ether to give 5-benzyloxy-2-[2-(N,N-dimethylamino)ethyl]-4-pyridone (3.45 g) as a powder.

IR (Nujol) : 1620 (sh), 1613 $cm^{-1}$

NMR (DMSO-$d_6$, δ) : 2.16 (6H, m), 2.55 (2H, m), 5.01 (2H, s), 6.13 (1H, s), 7.38 (5H, m), 7.40 (1H, s)

Preparation 5

(1) 5-Benzyloxy-2-(N,N-dimethylamino)methyl-4-pyridone (1.0 g) in methanol (15 ml) was subjected to catalytic reduction with 10% palladium on activated carbon (200 mg) at atmospheric pressure. After removal of catalyst, the solution was concentrated under reduced pressure to give 2-(N,N-dimethylamino)methyl-5-hydroxy-4-pyridone (0.96 g).

IR (Nujol) : 3300 (br), 1620 $cm^{-1}$
NMR (DMSO-$d_6$, δ) : 2.27 (6H, s), 3.43 (2H, s), 6.30 (1H, s), 7.40 (1H, s)

The following compounds were obtained according to a similar manner to that of Preparation 5(1).

(2) 5-Hydroxy-2-[2-(N,N-dimethylamino)ethyl]-4-pyridone

IR (Nujol) : 1640 (sh), 1630 $cm^{-1}$
NMR ($D_2O$, δ) : 2.50 (6H, m), 2.92 (2H, m), 6.48 (1H, s), 7.47 (1H, s)

(3) 5-Hydroxy-2-[2-(1-pyrrolidinyl)ethyl]-4-pyridone

IR (Nujol) : 1623 (sh), 1608 $cm^{-1}$
NMR ($D_2O$, δ) : 1.8-2.1 (4H, m), 2.7-3.3 (8H, m), 6.43 (1H, s), 7.36 (1H, s)

Example 1

To a mixture of benzhydryl 7β-[2-(5-amino-1,2,4-thiadiazol-3-yl)-2-methoxyiminoacetamido]-3-chloromethyl-3-cephem-4-carboxylate (syn isomer) (1.47 g), dichloromethane (5 ml) and anisole (1.4 ml) was added trifluoroacetic acid (5 ml) under ice-cooling with stirring. After being stirred for 30 minutes at the same temperature, the mixture was poured into diisopropyl ether (100 ml). The resulting precipitate was collected by filtration, washed with diisopropyl ether and dried under reduced pressure to give 7 -[2-(5-amino-1,2,4-thiadiazol-3-yl)-2-methoxyiminoacetamido]-3-chloromethyl-3-cephem-4-carboxylic acid trifluoroacetate (syn isomer). This compound was dissolved in N,N-dimethylformamide (15 ml). To the solution was added 2-(N,N-dimethylamino)methyl-5-hydroxy-4-pyridone (1.24 g). After being stirred for 5 hours at ambient temperature, the reaction mixture was poured into ethyl acetate (100 ml). The resulting precipitate was collected by filtration, washed with ethyl acetate. The precipitate was suspended in water (100 ml) and the suspension was adjusted to pH 2.0 with diluted hydrochloric acid. After removal of insoluble material by filtration, thee aqueous solution was subjected to column chromatography on Diaion HP-20 [Trademark : prepared by Mitsubishi Chemical Industries]. The column was washed with water and the elution was carried out with 30% aqueous methanol. The fractions containing desired product were combined and methanol was evaporated in vacuo. The resulting aqueous layer was lyophilized to give 7β-[2-(5-amino-1,2,4-thiadiazol-3-yl)-2-methoxyiminoacetamido]-3-[N,N-dimethyl- N-{(5-hydroxy-4-oxo-1,4-dihydropyridin-2-yl)methyl}ammonio]methyl-3-cephem-4-carboxylate (syn isomer) (0.28 g) as a powder.

IR (Nujol) : 3250 (br), 1765, 1650, 1600 $cm^{-1}$
NMR ($D_2O$-$NaHCO_3$, δ) : 3.0 (3H, br.s), 3.07 (3H, br.s), 3.40, 3.71 (2H, ABq, J=17Hz), 3.85-4.9 (4H, m), 4.05 (3H, s), 5.33 (1H, d, J=5Hz), 5.85 (1H, d, J=5Hz), 6.73 (1H, s), 7.70 (1H, s)

Example 2

(1) To a solution of 7β-[2-(5-amino-1,2,4-thiadiazol-3-yl)-2-(1-carboxy-1-methylethoxyimino)acetamido]-3-chloromethyl-3-cephem-4-carboxylic acid trifluoroacetate (syn isomer) (1.0 g) in-N,N-dimethylformamide (10 ml) was added 2-(N,N-dimethylamino)methyl-5-hydroxy-4-pyridone (1.09 g). After being stirred for 5 hours at ambient temperature, the mixture was poured into ethyl acetate (150 ml). The resulting precipitate was collected by filtration, washed with ethyl acetate and dried under reduced pressure. The precipitate was suspended in water (50 ml) at pH 2.0 and stirred for 30 minutes. After removal of insoluble material, the aqueous solution was subjected to column chromatography on Diaion HP-20. The column was washed with water and the elution was carried out with 30% aqueous methanol. The fractions containing desired product was combined and methanol was evaporated in vacuo. The resulting aqueous layer was lyophilized to give 7β-[2-(5-amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer) (0.30 g) as a powder.

IR (Nujol) : 3250 (br), 1770, 1670 (sh), 1600 $cm^{-1}$
NMR ($D_2O$-$NaHCO_3$, δ) : 1.56 (6H, s), 3.03 (3H, br. s), 3.10 (3H, br s), 3.44, 3.96 (2H, ABq, J=18Hz), 4.1-5.0 (4H, m), 5.37 (1H, d, J=5Hz), 5.88 (1H, d, J=5Hz), 6.76 (1H, s), 7.71 (1H, s)

The following compounds were obtained according to a similar manner to that of Example 2(1).

(2) 7β-[2-(5-Amino-1,2,4-thiadiazol-3-yl)-2-methoxyiminoacetamido]-3-[1-{2-(5-hydroxy-4-oxo-1,4-dihydropyridin-2-yl)ethyl}-1-pyrrolidinio]methyl-3-cephem-4-carboxylate (syn isomer)

IR (Nujol) : 3300 (br), 1765, 1663 (sh), 1620 $cm^{-1}$
NMR ($D_2O$-$NaHCO_3$, δ) : 2.05 (4H, m), 3.0-3.8 (10H, m), 4.07 (3H, s), 4.6-4.9 (2H, m), 5.18 (1H, d, J=5Hz), 5.80 (1H, d, J=5Hz), 6.45 (1H, s), 7.51 (1H, s)

(3) 7β-[2-(5-Amino-1,2,4-thiadiazol-3-yl)-2-(1-carboxy-1-methylethoxyimino)acetamido]-3-[1-{2-(5-hydroxy-4-oxo-1,4-dihydropyridin-2-yl)ethyl}-1-pyrrolidinio]methyl-3-cephem-4-carboxylate (syn isomer)

IR (Nujol) : 3300 (br), 1770, 1670-1620 (br) $cm^{-1}$
NMR ($D_2O$-$NaHCO_3$, δ): 1.53 (6H, s), 2.06 (4H, m), 3.0-3.8 (10H, m), 4.4-4.9 (2H, m), 5.10 (1H, d, J=5Hz), 5.80 (1H, d, J=5Hz), 6.43 (1H, s), 7.85 (1H, s)

(4) 7β-[2-(5-Amino-1,2,4-thiadiazol-3-yl)-2-(1-carboxy-1-methylethoxyimino)acetamido]-3-[N,N-dimethyl-N-{2-(5-hydroxy-4-oxo-1,4-dihydropyridin-2-yl)ethyl}ammonio]methyl-3-cephem-4-carboxylate (syn isomer)

IR (Nujol) : 3300 (br), 1775, 1670, 1615 $cm^{-1}$
NMR ($D_2O$-$NaHCO_3$, δ) : 1.55 (6H, s), 3.00 (3H, br.s), 3.15 (3H, br. s), 3.2-4.2 (4H, m), 4.6-4.9 (2H, m), 5.35 (1H, d, J=5Hz), 5.80 (1H, d, J=5Hz), 6.45 (1H, s), 7.54 (1H, s)

(5) 7β-[2-(5-Amino-1,2,4-thiadiazol-3-yl)-2-methoxyiminoacetamido]-3-[N,N-dimethyl-N-{2-(5-hydroxy-4-oxo-1,4-dihydropyridin-2-yl)ethyl}ammonio]methyl-3-cephem-4-carboxylate (syn isomer)

IR (Nujol) : 3250 (br), 1770, 1670(sh), 1610 $cm^{-1}$
NMR ($D_2O$-$NaHCO_3$, δ) : 3.06 (3H, br. s), 3.13 (3H, br.s), 3.2-4.2 (4H, m), 4.03 (3H, s), 4.6-4.9 (2H, m), 5.31 (1H, d, J=5Hz), 5.80 (1H, d, J=5Hz), 6.45 (1H, s), 7.50 (1H, s)

(6) 7β-[2-(5-Amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate hydrochloride (syn isomer)

IR (Nujol) : 3450-3150 (br), 2650, 1770, 1670, 1610 $cm^{-1}$

(7) Sulfuric acid salt of 7β-[2-(5-amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer)

IR (Nujol) : 3450-3150 (br), 2650 (br), 1780, 1692, 1615, 1558, 1529 $cm^{-1}$

Reference 1

To a suspension of 7β-[2-(2-formamidothiazol-4-yl)-2-tert-butoxycarbonylmethoxyiminoacetamido]-3-[N,N-dimethyl-N-{(5-hydroxy-4-oxo-1,4-dihydropyridin-2-yl)methyl}ammonio]methyl-3-cephem-4-carboxylate (syn isomer) (0.70 g) in methanol (35 ml) was added conc. hydrochloric acid (0.53 ml). After being stirred at ambient temperature, the reaction mixture was diluted with water (50 ml) and methanol was evaporated under reduced pressure. The resulting aqueous solution was adjusted to pH 1.0 and subjected to column chromatography on Diaion HP-20 (50 ml). The column was washed with water and the elution was carried out with 40% aqueous isopropanol. The fractions containing the object compound was lyophilized to give 7β-[2-(2-aminothiazol-4-yl)-2-tert-butoxycarbonylmethoxyiminoacetamido]-3-[N,N-dimethyl-N-{(5-hydroxy-4-oxo-1,4-dihydropyridin-2-yl)methyl}ammonio]methyl-3-cephem-4-carboxylate (syn isomer) (0.53 g).

| | |
|---|---|
| IR (Nujol) : | 3300 (br), 1750, 1730 (sh), 1667, 1608 $cm^{-1}$ |
| NMR ($D_2O$-$NaHCO_3$, δ) : | 1.50 (9H, s), 2.97 (3H, br.s), 3.06 (3H, br. s), 3.2-4.2 (4H, m), 4.36 (2H, br. s), 4.65 (2H, s), 5.35 (1H, d, J=5Hz), 5.86 (1H, d, J=5Hz), 6.73 (1H, s), 7.00 (1H, s), 7.73 (1H, s) |

Example 3

The following compounds were obtained according to a similar manner to that of Reference 1.

(1) 7β-[2-(5-Amino-1,2,4-thiadiazol-3-yl)-2-methoxyiminoacetamido]-3-[N,N-dimethyl-N-{(5-hydroxy-4-oxo-1,4-dihydropyridin-2-yl)methyl}ammonio]methyl-3-cephem-4-carboxylate (syn isomer)

IR (Nujol) : 3250 (br), 1765, 1650, 1600 $cm^{-1}$

(2) 7β-[2-(5-Amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer)

IR (Nujol) : 3250 (br.), 1770, 1670 (sh), 1600 $cm^{-1}$

(3) 7β-[2-(5-Amino-1,2,4,thiadiazol-3-yl)-2-methoxyiminoacetamido]-3-[1-{2-(5-hydroxy-4-oxo-1,4-dihydropyridin-2-yl)ethyl}-1-pyrrolidinio]methyl-3-cephem-4-carboxylate (syn isomer)

IR (Nujol) : 3300 (br.), 1765, 1663 (sh), 1620 $cm^{-1}$

(4) 7β-[2-(5-Amino-1,2,4-thiadiazol-3-yl)-2-(1-carboxy-1-methylethoxyimino)acetamido]-3-[1-{2-(5-hydroxy-4-oxo-1,4-dihydropyridin-2-yl)ethyl}-1-pyrrolidinio]methyl-3-cephem-4-carboxylate (syn isomer)

IR (Nujol) : 3300 (br), 1770, 1670-1620 (br.) $cm^{-1}$

(5) 7β-[2-(5-Amino-1,2,4-thiadiazol-3-yl)-2-(1-carboxy-1-methylethoxyimino)acetamido]-3-[N,N-dimethyl-N-{2-(5-hydroxy-4-oxo-1,4-dihydropyridin-2-yl)ethyl}ammonio]methyl-3-cephem-4-carboxylate (syn isomer)

IR (Nujol) : 3300 (br.), 1775, 1670, 1615 $cm^{-1}$

(6) 7β-[2-(5-Amino-1,2,4-thiadiazol-3-yl)-2-methoxyiminoacetamido]-3-[N,N-dimethyl-N-{2-(5-hydroxy-4-oxo-1,4-dihydropyridin-2-yl)ethyl}ammonio]methyl-3-cephem-4-carboxylate (syn isomer)

IR (Nujol) : 3250 (br), 1770, 1670 (sh), 1610 $cm^{-1}$

Reference 2

To a suspension of 7β-[2-(2-aminothiazol-4-yl)-2-tert-butoxycarbonylmethoxyiminoacetamido]-3-[N,N-dimethyl-N-{(5-hydroxy-4-oxo-1,4-dihydropyridin-2-yl)methyl}ammonio]methyl-3-cephem-4-carboxylate (syn isomer) (0.54 g) in anisole (2 ml) was dropwise added trifluoroacetic acid (8 ml) under ice-cooling with stirring. After being stirred at ambient temperature, the mixture was poured into diisopropyl ether (100 ml). The resulting precipitate was collected by filtration, washed with diisopropyl ether and dried under reduced pressure. The precipitate was dissolved in water (30 ml) at pH 7.0 and acidified to pH 1.0 with 6N hydrochloric acid. After removal of precipitated materials, the aqueous solution was subjected to column chromatography on Diaion HP-20 (50 ml). The column was washed with water and the elution was carried out with 30% aqueous methanol. The active fractions were collected and lyophilized to give 7β-[2-(2-aminothiazol-4-yl)-2-carboxymethoxyiminoacetamido]-3-[N,N-dimethyl-N-{(5-hydroxy-4-oxo-1,4-dihydropyridin-2-yl)methyl}ammonio]methyl-3-cephem-4-carboxylate (syn isomer) (0.28 g).

| | |
|---|---|
| IR (Nujol) : | 3250 (br), 1770, 1662 (sh), 1600 $cm^{-1}$ |
| NMR ($D_2O$-$NaHCO_3$, δ) : | 3.00 (3H, s), 3.07 (3H, s), 3.40, 3.94 (2H, ABq, J=18Hz), 4.1-5.1 (4H, m), 4.45 (2H, br. s), 4.60 (2H, s), 5.35 (1H, d, J=5Hz), 5.86 (1H, d, J=5Hz), 6.75 (1H, s), 6.93 (1H, s), 7.70 (1H, s) |

Example 4

The following compounds were obtained according to a similar manner to that of Reference 2.

(1) 7β-[2-(5-Amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer)

IR (Nujol) : 3250 (br), 1770, 1670 (sh), 1600 $cm^{-1}$

(2) 7β-[2-(5-Amino-1,2,4-thiadiazol-3-yl)-2-(1-carboxy-1-methylethoxyimino)acetamido]-3-[1-{2-(5-hydroxy-4-oxo-1,4-dihydropyridin-2-yl)ethyl}-1-pyrrolidinio]methyl-3-cephem-4-carboxylate (syn isomer)

IR (Nujol) : 3300 (br.), 1770, 1670-1620 (br)

(3) 7β-[2-(5-Amino-1,2,4-thiadiazol-3-yl)-2-(1-carboxy-1-methylethoxyimino)acetamido]-3-[N,N-dimethyl-N-{2-(5-hydroxy-4-oxo-1,4-dihydropyridin-2-yl)ethyl}ammonio]methyl-3-cephem-4-carboxylate (syn isomer)

IR (Nujol) : 3300 (br.), 1775, 1670, 1615 $cm^{-1}$

Preparation 6

To a suspension of 5-benzyloxy-2-hydroxymethyl-4-pyridone (33 g) in benzene (500 ml) was added thionyl chloride (28.4 ml) at ambient temperature under stirring. After being stirred at the same temperature for 30 minutes, the mixture was refluxed for 4 hours. The resulting mixture was cooled to ambient temperature. The precipitate was collected by filtration, washed with benzene and dried under reduced pressure to give 5-benzyloxy-2-chloromethyl-4-pyridone (41.5 g).

IR (Nujol) : 1608, 1585 $cm^{-1}$
NMR (DMSO-$d_6$, δ) : 5.00 (2H, s), 5.30 (2H, s), 7.4 (5H, m), 7.56 (1H, s), 8.43 (1H, s)

Preparation 7

A suspension of benzhydryl 7β-amino-3-chloromethyl-3-cephem-4-carboxylate hydrochloride (36.11 g) in a mixture of ethyl acetate (900 ml) and cold water (360 ml) was adjusted to pH 7 with a saturated aqueous solution of potassium carbonate. The separated organic layer was washed with brine, dried over magnesium sulfate and evaporated in vacuo. The residue was pulverized with diisopropyl ether to give the powder (34.40 g). The powder (34.40 g) was added portionwise at 5°C to acetic formic anhydride prepared from formic acid (15.27 g) and acetic anhydride (33.89 g). The mixture was warmed to room temperature and stirred for 1.8 hours at the same temperature. The mixture was poured into a mixture of ethyl acetate (1 ℓ) and cold water (400 ml) and adjusted to pH 7 with 20% aqueous sodium hydroxide solution under cooling. The separated organic layer was washed with water and brine, dried over magnesium sulfate and concentrated to 100 ml. The residual solution was poured into a mixture of diisopropyl ether (1 ℓ) and hexane (1 ℓ) and the resulting precipitates were collected by filtration to give benzhydryl 7β-formamido-3-chloromethyl-3-cephem-4-carboxylate (29.07 g).

IR (Nujol) : 1780, 1720, 1675 $cm^{-1}$
NMR (DMSO-$d_6$, δ) : 3.55 and 3.79 (2H, ABq, J=18Hz), 4.53 (2H, s), 5.23 (1H, d, J=5Hz), 5.89 (1H, dd, J=8 and 5Hz), 6.96 (1H, s), 7.2-7.6 (10H, m), 8.15 (1H, s), 9.10 (1H, d, J=8Hz)

Preparation 8

To a solution of benzhydryl 7β-formamido-3-chloromethyl-3-cephem-4-carboxylate (27.34 g) in a mixture of dichloromethane (137 ml) and anisole (27 ml) was added dropwise trifluoroacetic acid (54 ml) and the mixture was stirred at 5°C for 1.2 hours. The mixture was added dropwise to a cooled mixture of diisopropyl ether (2 ℓ) and hexane (2 ℓ) and the resulting precipitates were collected by filtration, washed with a mixture of diisopropyl ether and hexane to give 7β-formamido-3-chloromethyl-3-cephem-4-carboxylic acid (14.03 g).

IR (Nujol) : 1775, 1665, 1520 $cm^{-1}$
NMR (DMSO-$d_6$, δ) : 3.49 and 3.74 (2H, ABq, J=18Hz), 4.55 (2H, s), 5.15 (1H, d, J=5Hz), 5.78 (1H, dd, J=8 and 5Hz), 8.12 (1H, s), 9.06 (1H, d, J=8Hz)

Preparation 9

2-(N,N-Dimethylamino)methyl-5-hydroxy-4-pyridone (15.85 g) was dissolved in N,N-dimethylformamide (238 ml) by addition of sodium 2-ethylhexanoate (10.44 g). To the resulting solution was added dropwise a cooled solution of 7β-formamido-3-chloromethyl-3-cephem-4-carboxylic acid (13.04 g) at 5°C and the mixture was stirred for 2 hours at the same temperature. The mixture was added dropwise to a mixture of ethyl acetate (2.5 ℓ) and diisopropyl ether (2.5 ℓ) and the resulting precipitates were collected by filtration,washed three times with a mixture of ethyl acetate and diisopropyl ether (1:1) and dried in vacuo. The powder was poured into cold water (300 ml) and the mixture was adjusted to pH 3.0 with 1N hydrochloric acid. After the insoluble materials were filtered off, the filtrate was chromatographed on Diaion HP-20 (1300 ml) at 50°C and the elution was carried out with 10% aqueous isopropyl alcohol. The eluate was lyophilized to give 7β-formamido-3-[N,N-dimethyl-N-{(5-hydroxy-4-oxo-1,4-dihydropyridin-2-yl)methyl}ammonio]methyl-3-cephem-4-carboxylate (8.31 g).

IR (Nujol) : 1775, 1670, 1610, 1565, 1515 $cm^{-1}$

NMR ($D_2O$, δ) : 3.05 and 3.14 (6H, s x 2), 3.47 and 3.99 (2H, ABq, J=18Hz), 4.45-4.55 (2H, m), 4.40 and 4.90 (2H, ABq, J=14Hz), 5.31 (1H, d, J=5Hz), 5.80 (1H, d, J=5Hz), 6.84 (1H, s), 7.81 (1H, s), 8.24 (1H, s)

Preparation 10

To a cooled suspension of 7β-formamido-3-[N,N-dimethyl-N-{(5-hydroxy-4-oxo-1,4-dihydropyridin-2-yl)methyl}ammonio]methyl-3-cephem-4-carboxylate (1.225 g) in methanol (12 ml) was added dropwise conc. hydrochloric acid (0.83 ml) at 10°C. The mixture was warmed to room temperature and stirred for 2.5 hours. The mixture was poured into ethyl acetate (300 ml) and the precipitates were collected by filtration, washed with ethyl acetate and diisopropyl ether and dried in vacuo. The powder was dissolved in cold water (10 ml) and the resulting solution was chromatographed on Diaion HP-20 (10 ml) at 5°C and the elution was carried out with cold water. To the eluate (24 ml) was added dropwise isopropyl alcohol (12 ml) under cooling and the mixture was allowed to stand overnight in a refrigerator. The resultant crystal was collected by filtration, washed with a cold mixture of isopropyl alcohol and water (10:1) and cold isopropyl alcohol, and dried in vacuo to give 7β-amino-3-[N,N-dimethyl-N-{(5-hydroxy-4-oxo-1,4-dihydropyridin-2-yl)methyl}ammonio]methyl-3-cephem-4-carboxylate dihydrochloride (440 mg).

IR (Nujol) : 3350, 1810, 1790, 1640, 1520 $cm^{-1}$

NMR ($D_2O$ + $NaHCO_3$, δ) : 3.04 and 3.14 (6H, s x 2), 3.48 and 3.99 (2H, ABq, J=18Hz), 4.13 and 4.72 (2H, ABq, J=14Hz), 4.45-4.55 (2H, m), 4.94 (1H, d, J=5Hz), 5.29 (1H, d, J=5Hz), 6.85 (1H, s), 7.82 (1H, s)

Preparation 11

To a cooled solution of 7β-formamido-3-[N,N-dimethyl-N-{(5-hydroxy-4-oxo-1,4-dihydropyridin-2-yl)methyl}ammonio]methyl-3-cephem-4-carboxylate (4.084 g) in formic acid (8.1 ml) was added dropwise conc. hydrochloric acid (2.5 ml). The mixture was warmed to room temperature and stirred for 2.5 hours. The mixture was added dropwise to ethyl acetate (400 ml) and the supernatant was decanted. The residual oil was dissolved in methanol (50 ml) and the solution was poured into ethyl acetate (600 ml). The resulting precipitates were collected by filtration, washed with ethyl acetate and diisopropyl ether and dried in vacuo. The powder was dissolved in cold water (28 ml) and chromatographed on Diaion HP-20 (28 ml) at 5°C, and the elution was carried out with water. To the eluate (30 ml) was added dropwise cold isopropyl alcohol (19 ml) under cooling and the mixture was stirred at 5°C for an hour. The resultant crystal was collected by filtration, washed with a cooled mixture of isopropyl alcohol and water (10:1) and cold isopropyl alcohol, and dried in vacuo to give 7β-amino-3-[N,N-dimethyl-N-{(5-hydroxy-4-oxo-1,4-dihydropyridin-2-yl)methyl}ammonio]methyl-3-cephem-4-carboxylate dihydrochloride (728 mg).

IR (Nujol) : 3350, 1810, 1790, 1640, 1520 $cm^{-1}$

NMR ($D_2O$ + $NaHCO_3$, δ) : 3.04 and 3.14 (6H, s x 2), 3.48 and 3.99 (2H, ABq, J=18Hz), 4.13 and 4.72 (2H, ABq, J=14Hz), 4.45-4.55 (2H, m), 4.94 (1H, d, J=5Hz), 5.29 (1H, d, J=5Hz), 6.85 (1H, s), 7.82 (1H, s)

Example 5

7β-amino-3-[N,N-dimethyl-N-{(5-hydroxy-4-oxo-1,4-dihydropyridin-2-yl)methyl}ammonio]methyl-3-cephem-4-carboxylate dihydrochloride (181 mg) was suspended in a mixture of water (7.2 ml) and tetrahydrofuran (3.6 ml) and

adjusted to pH 5 with a saturated aqueous solution of sodium bicarbonate. To the resulting solution was added portionwise a solution of 2-(5-amino-1,2,4-thiadiazol-3-yl)-2-(1-carboxy-1-methylethoxyimino)acetic methanesulfonic anhydride (syn isomer) (169 mg) in tetrahydrofuran (0.5 ml) at 15°C and the mixture was stirred for an hour at 15°C and pH 4.0-6.0. After tetrahydrofuran was evaporated in vacuo, water (30 ml) was added to the residue. The aqueous solution was adjusted to pH 1.0 with 1N hydrochloric acid and filtered to remove the insoluble materials. The filtrate was chromatographed on Diaion HP-20 (15 ml) and the elution was carried out with 15% aqueous isopropyl alcohol. The eluate was lyophilized to give 7β-[2-(5-amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer) (124 mg).

IR (Nujol) : 3250 (br), 1770, 1670 (sh), 1600 $cm^{-1}$
NMR ($D_2O$-$NaHCO_3$, δ) : 1.56 (6H, s), 3.03 (3H, br s), 3.10 (3H, br s), 3.44, 3.96 (2H, ABq, J=18Hz), 4.1-5.0 (4H, m), 5.37 (1H, d, J=5Hz), 5.88 (1H, d, J=5Hz), 6.76 (1H, s), 7.71 (1H, s)

Example 6

7β-Amino-3-[N,N-dimethyl-N-{(5-hydroxy-4-oxo-1,4-dihydropyridin-2-yl)methyl}ammonio]methyl-3-cephem-4-carboxylate dihydrochloride (181 mg) was suspended in tetrahydrofuran (9.1 ml). To the suspension was added N-(trimethylsilyl)acetamide (788 mg), and the mixture was stirred at 35°C for 30 minutes and cooled to 5°C. To the resulting solution was added a solution of 2-(5-amino-1,2,4-thiadiazol-3-yl)-2-(1-carboxy-1-methylethoxyimino)acetic methanesulfonic anhydride (syn isomer) (169 mg) in tetrahydrofuran (0.5 ml). The mixture was warmed to 20°C and stirred for 1.2 hours. The mixture was poured into ethyl acetate (200 ml) and the resulting precipitates were collected by filtration, washed with ethyl acetate and diisopropyl ether and dried in vacuo. The powder was suspended in water (40 ml) and adjusted to pH 1 with 1N hydrochloric acid. After the insoluble materials were removed by filtration, the filtrate was chromatographed on Diaion HP-20 (15 ml) and the elution was carried out with 15% aqueous isopropyl alcohol. The eluate was lyophilized to give 7β-[2-(5-amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer) (120 mg).

IR (Nujol) : 3250 (br), 1770, 1670 (sh), 1600 $cm^{-1}$

Preparation 12

To a solution of benzhydryl 7β-amino-3-chloromethyl-3-cephem-4-carboxylate hydrochloride (22.6 g) and N-trimethylsilylacetamide (25.0 g) in tetrahydrofuran (250 ml) was added 2-(5-amino-1,2,4-thiadiazol-3-yl)-2-carboxymethoxyiminoacetic methanesulfonic anhydride (syn isomer) (19.8 g) at 0-5°C under stirring. The stirring was continued for an hour at the same temperature. The reaction mixture was poured into a mixture of ethyl acetate (500 ml) and water (500 ml). The organic layer was separated, washed with water twice and a saturated aqueous solution of sodium chloride, dried over magnesium sulfate and then concentrated in vacuo. To the residue was added diethyl ether and then the resulting precipitates were collected by filtration to give benzhydryl 7β-[2-(5-amino-1,2,4-thiadiazol-3-yl)-2-carboxymethoxyiminoacetamido]-3-chloromethyl-3-cephem-4-carboxylate (syn isomer) (17.2 g).

NMR (DMSO-$d_6$, δ) : 3.57, 3.70 (2H, ABq, J=18Hz), 4.42 (2H, s), 4.63 (2H, s), 5.22 (1H, d, J=5Hz), 5.90 (1H, dd, J=5Hz, J=8Hz), 6.92 (1H, s), 7.17-7.53 (5H, m), 8.07 (2H, br s), 9.52 (1H, d, J=8Hz)

Preparation 13

To a solution of trifluoroacetic acid (20 ml) and anisole (10 ml) in methylene chloride (50 ml) was added benzhydryl 7β-[2-(5-amino-1,2,4-thiadiazol-3-yl)-2-carboxymethoxyiminoacetamido]-3-chloromethyl-3-cephem-4-carboxylate (syn isomer) (7.0 g) at 0 ~ -5°C under stirring. The stirring was continued for 2 hours at the same temperature. The reaction mixture was poured into a cold mixture of diisopropyl ether (400 ml) and n-hexane (200 ml). The resulting precipitates were collected by filtration to give 7β-[2-(5-amino-1,2,4-thiadiazol-3-yl)-2-carboxymethoxyiminoacetamido]-3-chloromethyl-3-cephem-4-carboxylic acid (syn isomer) (5.5 g).

IR (Nujol) : 3300, 1770, 1700, 1670, 1610, 1510 $cm^{-1}$
NMR (DMSO-$d_6$, δ) : 3.53, 3.73 (2H, ABq, J=18Hz), 4.55 (2H, s), 4.65 (2H, s), 5.17 (1H, d, J=5Hz), 5.83 (1H, dd, J=5Hz, J=8Hz), 8.10 (2H, br s), 9.48 (1H, d, J=8Hz)

Example 7

To 1N hydrochloric acid was added 7β-[2-(5-amino-1,2,4-thiadiazol-3-yl)-2-(1-carboxy-1-methylethoxyimino)-acetamido]-3-[N,N-dimethyl-N-{(5-hydroxy-4-oxo-1,4-dihydropyridin-2-yl)methyl}ammonio]methyl-3-cephem-4-carboxylate (syn isomer) (2.2 g). The mixture was stirred under ice-cooling for 3 hours. The resulting precipitate was collected by filtration, washed with a small amount of cold water and dried under reduced pressure to give 7β-[2-(5-amino-1,2,4-thiadiazol-3-yl)-2-(1-carboxy-1-methylethoxyimino)acetamido]-3-{N,N-dimethyl-N-{(5-hydroxy-4-oxo-1,4-dihydropyridin-2-yl)methyl}ammonio]methyl-3-cephem-4-carboxylate hydrochloride (syn isomer) (1.1 g).

IR (Nujol) : 3450-3150 (br), 2650, 1770, 1670, 1610 $cm^{-1}$

Example 8

To a mixture of water (22 ml) and acetone (22 ml) was added 7β-[2-(5-amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer) (2.20 g). To the resulting solution was added 97% sulfuric acid (0.699 g). The solution was stirred for 6.5 hours at 25 to 30°C. The resulting crystals were collected by filtration, washed with a mixture of water (9 ml) and acetone (9 ml) and dried under reduced pressure to give sulfuric acid salt (2.17 g) of 7β-[2-(5-amino-1,2,4-thiadiazol-3-yl)-2-(1-carboxy-1-methylethoxyimino)acetamido]-3-[N,N-dimethyl-N-{(5-hydroxy-4-oxo-1,4,dihydropyridin-2-yl)methyl}ammonio]methyl-3-cephem-4-carboxylate (syn isomer).

mp : 145 (dec.)
IR (Nujol) : 3450-3150 (br), 2650 (br), 1780, 1692, 1615, 1558, 1529$cm^{-1}$

Example 9

The following compounds were obtained according to similar manner to those of Examples 5 and 6.

(1) 7β-[2-(5-Amino-1,2,4-thiadiazol-3-yl)-2-methoxyiminoacetamido]-3-[N,N-dimethyl-N-{(5-hydroxy-4-oxo-1,4-dihydropyridin-2-yl)methyl}ammonio]methyl-3-cephem-4-carboxylate (syn isomer)

IR (Nujol) : 3250 (br), 1765, 1650, 1600 $cm^{-1}$

(2) 7β-[2-(5-Amino-1,2,4-thiadiazol-3-yl)-2-methoxyiminoacetamido]-3-[1-{2-(5-hydroxy-4-oxo-1,4-dihydropyridin-2-yl)ethyl}-1-pyrrolidinio]methyl-3-cephem-4-carboxylate (syn isomer)

IR (Nujol) : 3300 (br), 1765, 1663 (sh), 1620 $cm^{-1}$

(3) 7β-[2-(5-Amino-1,2,4-thiadiazol-3-yl)-2-(1-carboxy-1-methylethoxyimino)acetamido]-3-[1-{2-(5-hydroxy-4-oxo-1,4-dihydropyridin-2-yl)ethyl}-1-pyrrolidinio]methyl-3-cephem-4-carboxylate (syn isomer)

IR (Nujol) : 3300 (br), 1770, 1670~1620 (br) $cm^{-1}$

(4) 7β-[2-(5-Amino-1,2,4-thiadiazol-3-yl)-2-(1-carboxy-1-methylethoxyimino)acetamido]-3-[N,N-dimethyl-N-{2-(5-hydroxy-4-oxo-1,4-dihydropyridin-2-yl)ethyl}ammonio]methyl-3-cephem-4-carboxylate (syn isomer)

IR (Nujol) : 3300 (br), 1775, 1670, 1615 $cm^{-1}$

(5) 7β-[2-(5-Amino-1,2,4-thiadiazol-3-yl)-2-methoxyiminoacetamido]-3-[N,N-dimethyl-N-{2-(5-hydroxy-4-oxo-1,4-dihydropyridin-2-yl)ethyl}ammonio]methyl-3-cephem-4-carboxylate (syn isomer)

IR (Nujol) : 3250 (br), 1770, 1670 (sh), 1610 $cm^{-1}$

(6) 7β-[2-(5-Amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate hydrochloride (syn isomer)

IR (Nujol) : 3450-3150 (br), 2650, 1770, 1670, 1610 $cm^{-1}$

(7) Sulfuric acid salt of 7β-[2-(5-amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer)

IR (Nujol) : 3450-3150 (br), 2650 (br), 1780, 1692, 1615, 1558, 1529 $cm^{-1}$

## Claims

**Claims for the following Contracting States : AT, BE, CH, DE, FR, GB, IT, LI, LU, NL, SE**

1. A compound of the formula :

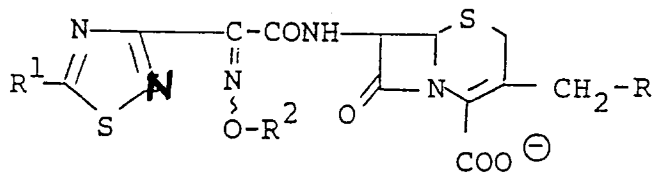

wherein

$R^1$ is amino or a protected amino group,
$R^2$ is $C_1$-$C_6$ alkyl, $C_2$-$C_6$ alkenyl, carboxy($C_1$-$C_6$)alkyl or protected carboxy($C_1$-$C_6$)alkyl, and
R is a group of the formula :

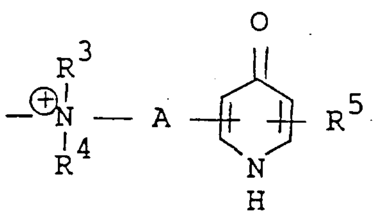

(in which $R^3$ and $R^4$ are each $C_1$-$C_6$ alkyl, or
$R^3$ and $R^4$ are linked together to form $C_3$-$C_6$ alkylene,
A is $C_1$-$C_6$ alkylene, and
$R^5$ is hydroxy or a protected hydroxy group),

and a pharmaceutically acceptable salt thereof.

2. A compound of claim 1, wherein

$R^1$ is amino or acylamino and
$R^2$ is $C_1$-$C_6$ alkyl, $C_1$-$C_6$ alkenyl, carboxy($C_1$-$C_6$)alkyl or esterified carboxy($C_1$-$C_6$)alkyl.

3. A compound of claim 2, wherein

$R^1$ is amino or $C_1$-$C_6$ alkanoylamino.

4. A compound of claim 3, wherein R is a group of the formula :

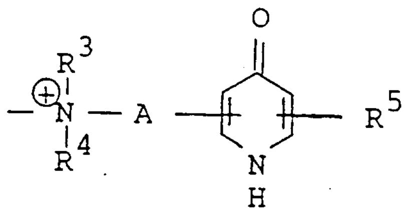

(in which $R^3$ and $R^4$ are each $C_1$-$C_6$ alkyl,

$R^5$ is hydroxy and
A is $C_1$-$C_6$ alkylene).

**5.** A compound of claim 4, wherein $R^1$ is amino and

$R^2$ is carboxy($C_1$-$C_6$)alkyl.

**6.** A compound of claim 5, which is 7β-[2-(5-amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer).

**7.** A compound of claim 5, which is sulfuric acid salt of 7β-[2-(5-amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer).

**8.** A process for preparing a compound of claim 1 or a salt thereof, which comprises

(1) reacting a compound of the formula :

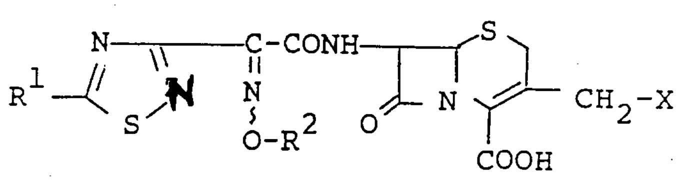

wherein

$R^1$, and $R^2$ are each as defined above and
X is an acid residue,

or a salt thereof with a compound of the formula :

$R^a$

wherein

$R^a$ is a compound of the formula :

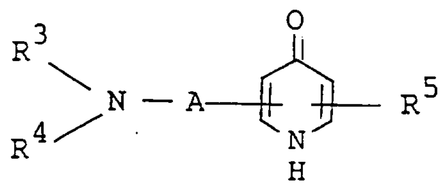

(in which $R^3$, $R^4$, $R^5$ and A are each as defined above), or a salt thereof to give a compound of the formula :

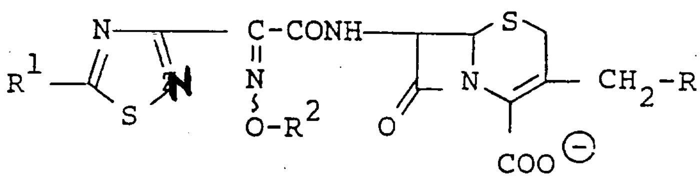

wherein

$R$, $R^1$ and $R^2$ are each as defined above,

or a salt thereof, or

(2) subjecting a compound of the formula :

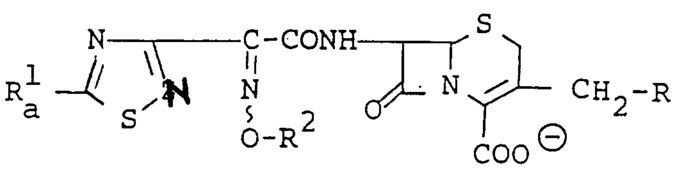

wherein

$R$, and $R^2$ are each as defined above and

$R^1_a$ is a protected amino group,

or a salt thereof to elimination reaction of the amino protective group to give a compound of the formula :

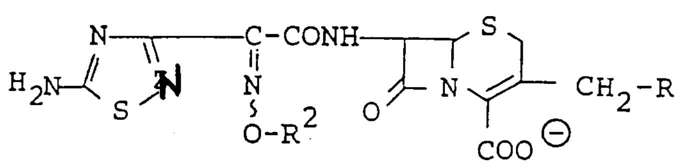

wherein R, and $R^2$ are each as defined above, or a salt thereof, or

(3) subjecting a compond of the formula :

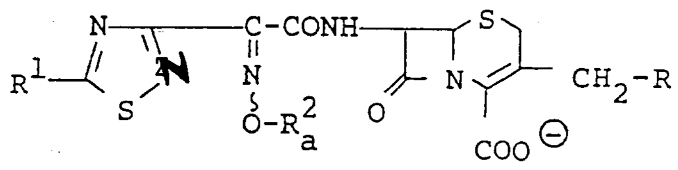

wherein

R, and $R^1$ are each as defined above and
$R^2_a$ is protected carboxy($C_1$-$C_6$)alkyl,

or a salt thereof to elimination reaction of the carboxy protective group to give a compound of the formula :

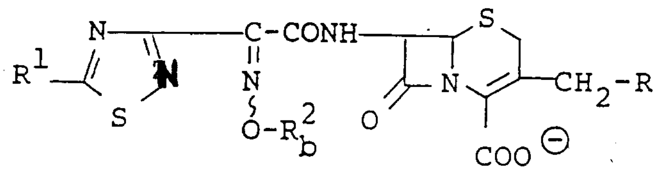

wherein

R, and $R^1$ are each as defined above and
$R^2_b$ is carboxy($C_1$-$C_6$)alkyl,

or a salt thereof, or

(4) reacting a compound of the formula :

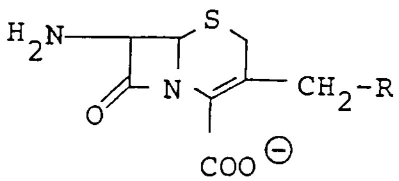

wherein R is as defined above,
or its reactive derivative at the amino group or a salt thereof with a compound of the formula:

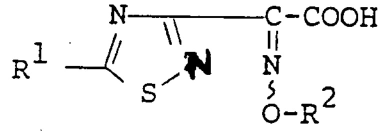

wherein $R^1$ and, $R^2$ are each as defined above, or its reactive derivative at the carboxy group or a salt thereof to give a compound of the formula :

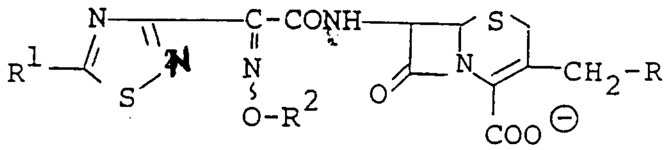

wherein

R, $R^1$, and $R^2$ are each as defined above,

or a salt thereof.

**9.** A compound of the formula :

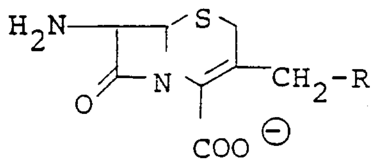

wherein R is a group of the formula :

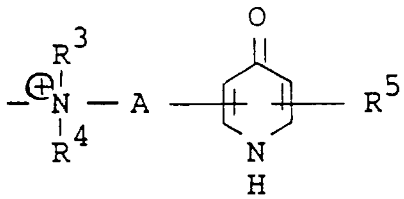

(in which $R^3$ and $R^4$ are each $C_1$-$C_6$ alkyl, or

| | |
|---|---|
| $R^3$ and $R^4$ | are linked together to form $C_3$-$C_6$ alkylene, |
| A | is $C_1$-$C_6$ alkylene, and |
| $R^5$ | is hydroxy or a protected hydroxy group) |

or a salt thereof.

**10.** A process for preparing a compound of claim 9 or a salt thereof, which comprises subjecting a compound of the formula:

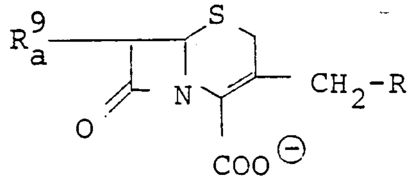

wherein

| | |
|---|---|
| R | is as defined above and |
| $R^9_a$ | is a protected amino group, |

or a salt thereof to elimination reaction of the amino protective group.

11. A pharmaceutical composition which comprises, as an active ingredient, a compound of claim 1 or a pharmaceutically acceptable salt thereof in admixture with pharmaceutically acceptable carriers.

12. A compound of claim 1 or pharmaceutically acceptable salts thereof for use as a medicament.

13. A compound of claim 1 or pharmaceutically acceptable salts thereof for use in treating or preventing infectious diseases.

14. A use of a compound of claim 1 or a pharmaceutical salt thereof for manufacture of medicament for treating or preventing infectious diseases.

**Claims for the following Contracting States : ES, GR**

1. A process for preparing a compound of the formula :

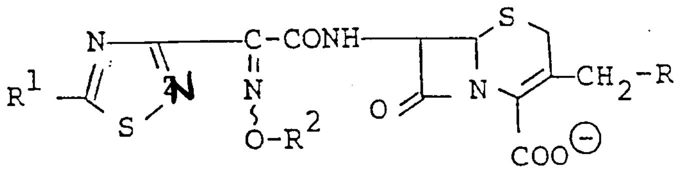

(I)

wherein

| | |
|---|---|
| $R^1$ | is amino or a protected amino group, |
| $R^2$ | is $C_1$-$C_6$ alkyl, $C_2$-$C_6$ alkenyl, carboxy($C_1$-$C_6$)alkyl or protected carboxy($C_1$-$C_6$)alkyl |
| R | is a group of the formula : |

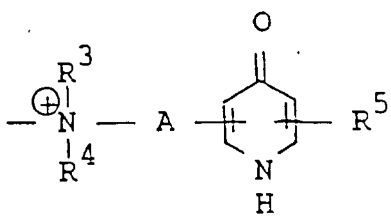

| | |
|---|---|
| | (in which $R^3$ and $R^4$ are each $C_1$-$C_6$ alkyl, or |
| $R^3$ and $R^4$ | are linked together to form $C_3$-$C_6$ alkylene, |
| A | is $C_1$-$C_6$ alkylene, and |
| $R^5$ | is hydroxy or a protected hydroxy group), or a salt thereof, |

which comprises

(1) reacting a compound of the formula :

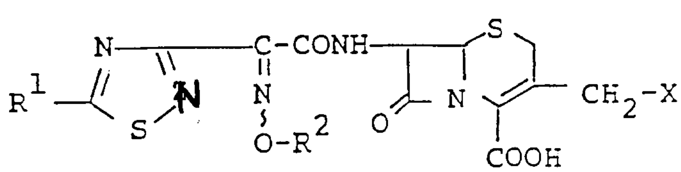

wherein

| | |
|---|---|
| $R^1$ and, $R^2$ | are each as defined above and |
| X | is an acid residue, |

or a salt thereof with a compound of the formula :

$R^a$

wherein

$R^a$ is a compound of the formula :

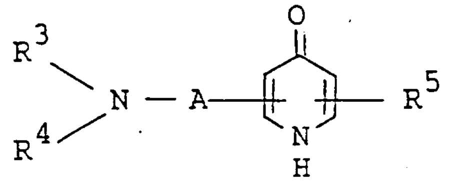

(in which $R^3$, $R^4$, $R^5$ and A are each as defined above), or a salt thereof to give a compound of the formula :

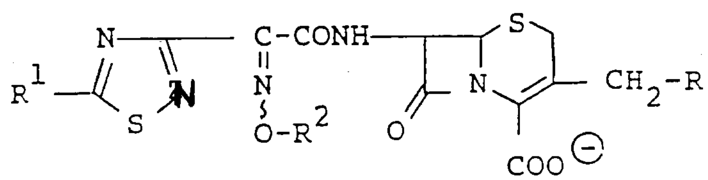

wherein R, $R^1$, and $R^2$ are each as defined above,

or a salt thereof, or

(2) subjecting a compound of the formula :

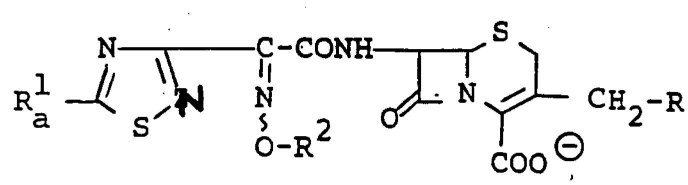

wherein

| | |
|---|---|
| R, and $R^2$ | are each as defined above and |
| $R^1_a$ | is a protected amino group, |

or a salt thereof to elimination reaction of the amino protective group to give a compound of the formula :

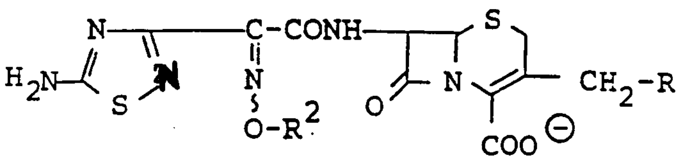

wherein R, and $R^2$ are each as defined above, or a salt thereof, or

(3) subjecting a compond of the formula :

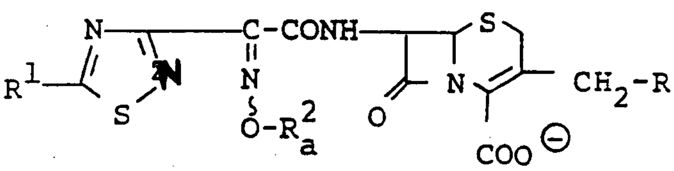

wherein

R, and $R^1$ are each as defined above and
$R^2_a$ is protected carboxy($C_1$-$C_6$)alkyl,

or a salt thereof to elimination reaction of the carboxy protective group to give a compound of the formula :

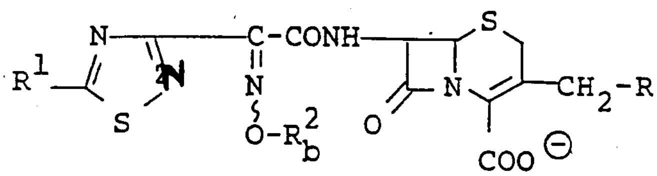

wherein

R, and $R^1$ are each as defined above and
$R^2_b$ is carboxy($C_1$-$C_6$)alkyl,

or a salt thereof, or

(4) reacting a compound of the formula :

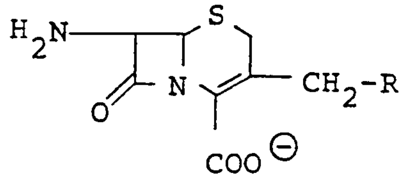

wherein R is as defined above,
or its reactive derivative at the amino group or a salt thereof with a compound of the formula:

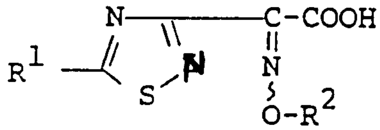

wherein $R^1$, and $R^2$ are each as defined above, or its reactive derivative at the carboxy group or a salt thereof to give a compound of the formula :

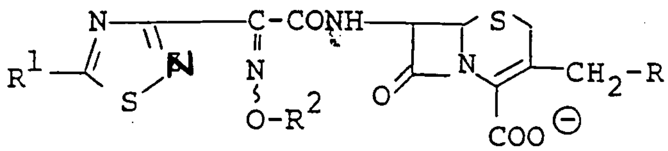

wherein

$R$, $R^1$, and $R^2$ are each as defined above,

or a salt thereof.

**2.** A process of claim 1, wherein

$R^1$ is amino or acylamino and
$R^2$ is $C_1$-$C_6$ alkyl, $C_1$-$C_6$ alkenyl, carboxy($C_1$-$C_6$)alkyl or esterified carboxy($C_1$-$C_6$)alkyl.

**3.** A process of claim 2, wherein $R^1$ is amino or $C_1$-$C_6$ alkanoylamino.

**4.** A process of claim 3, wherein R is a group of the formula :

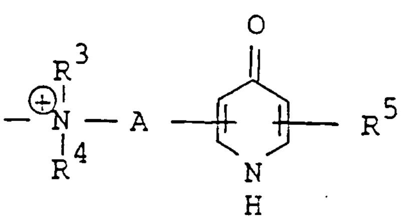

(in which $R^3$ and $R^4$ are each $C_1$-$C_6$ alkyl,

$R^5$ is hydroxy and
A is $C_1$-$C_6$ alkylene).

**5.** A process of claim 4, wherein

$R^1$ is amino and

$R^2$ is carboxy($C_1$-$C_6$)alkyl.

6. A process of claim 5 for preparing, 7β-[2-(5-amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer).

7. A process of claim 5 for preparing the, sulfuric acid salt of 7β-[2-(5-amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylate (syn isomer).

8. A process for preparing a compound of the formula :

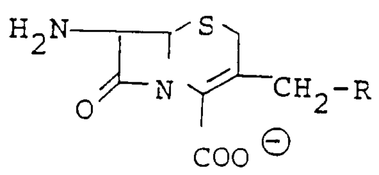

wherein

R is a group of the formula :

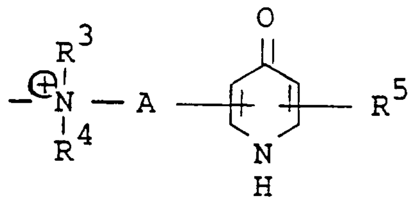

(in which $R^3$ and $R^4$ are each $C_1$-$C_6$ alkyl, or

$R^3$ and $R^4$ are linked together to form $C_3$-$C_6$ alkylene,

A is $C_1$-$C_6$ alkylene, and

$R^5$ is hydroxy or a protected hydroxy group)

or a salt thereof,
which comprises subjecting a compound of the formula:

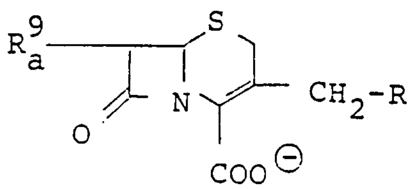

wherein

R is as defined above and

$R^9_a$ is a protected amino group,

or a salt thereof to elimination reaction of the amino protective group.

9. A process for the preparation of a pharmaceutical composition which is characterised by bringing a compound of formula I, or a non-toxic salt thereof, produced by a process claimed in claim 1, into pharmaceutically acceptable form by admixture or presentation of said compound with a pharmaceutically acceptable diluent or carrier.

**Patentansprüche**

**Patentansprüche für folgende Vertragsstaaten : AT, BE, CH, DE, FR, GB, IT, LI, LU, NL, SE**

1. Eine Verbindung der Formel

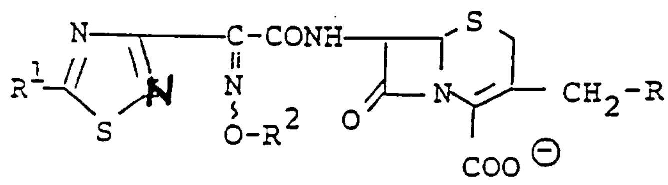

wobei $R^1$ Amino oder eine geschützte Aminogruppe ist,
$R^2$ $(C_1\text{-}C_6)$Alkyl, $(C_2\text{-}C_6)$Alkenyl, Carboxy$(C_1\text{-}C_6)$alkyl oder geschütztes Carboxy$(C_1\text{-}C_6)$alkyl ist und R eine Gruppe der Formel ist:

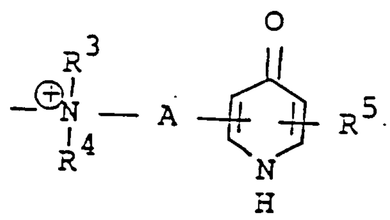

(wobei $R^3$ und $R^4$ jeweils$(C_1\text{-}C_6)$Alkyl ist, oder
$R^3$ und $R^4$ unter Bildung von$(C_3\text{-}C_6)$Alkylen aneinander gebunden sind, A $(C_1\text{-}C_6)$ Alkylen ist, und
$R^5$ Hydroxy oder eine geschützte Hydroxygruppe ist), und ein pharmazeutisch verträgliches Salz davon.

2. Eine Verbindung aus Anspruch 1,
wobei $R^1$ Amino oder Acylamino ist und
$R^2$ $(C_1\text{-}C_6)$ Alkyl, $(C_1\text{-}C_6)$ Alkenyl, Carboxy$(C_1\text{-}C_6)$alkyl oder verestertes Carboxy$(C_1\text{-}C_6)$alkyl ist.

3. Eine Verbindung aus Anspruch 2,
wobei $R^1$ Amino oder $(C_1\text{-}C_6)$ Alkanoylamino ist.

4. Eine Verbindung aus Anspruch 3,
wobei R eine Gruppe der Formel ist:

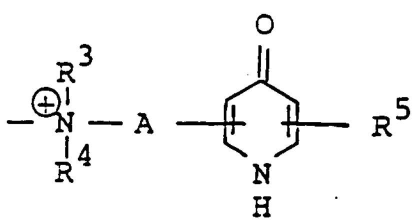

(wobei $R^3$ und $R^4$ jeweils $(C_1\text{-}C_6)$Alkyl sind,
$R^5$ Hydroxy ist und
A $(C_1\text{-}C_6)$ Alkylen ist).

5. Eine Verbindung aus Anspruch 4,
wobei $R^1$ Amino ist und
$R^2$ Carboxy$(C_1\text{-}C_6)$alkyl ist.

6. Eine Verbindung aus Anspruch 5, die 7β-[2-(5-Amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylat (syn-Isomer) ist.

7. Eine Verbindung aus Anspruch 5, die das Schwefelsäuresalz von 7β-[2-(5-Amino-1,2,4-thiadiazol-3-yl)-2-(1-carboxyl-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylat (syn-Isomer) ist.

8. Ein Verfahren zur Herstellung einer Verbindung aus Anspruch 1, oder eines Salzes davon, welches umfaßt

(1) Umsetzung einer Verbindung der Formel:

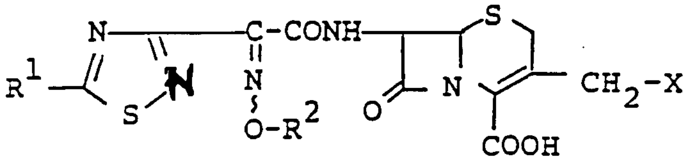

wobei $R^1$ und $R^2$ jeweils wie oben definiert sind und X ein Säurerest ist, oder eines Salzes davon mit einer Verbindung der Formel:

$R^a$

wobei $R^a$ eine Verbindung der Formel ist:

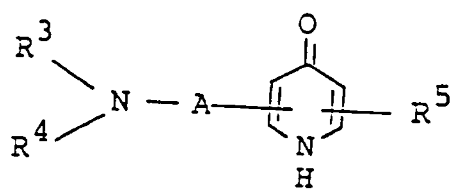

(wobei $R^3$, $R^4$, $R^5$ und A jeweils wie oben definiert sind), oder eines Salzes davon zur Herstellung einer Verbindung der Formel:

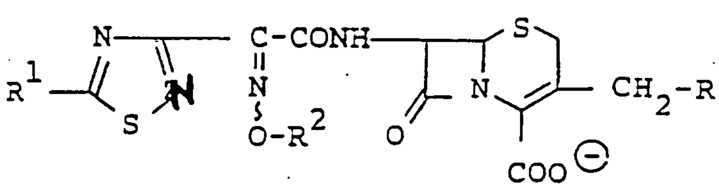

worin R, $R^1$ und $R^2$ jeweils wie oben definiert sind, oder eines Salzes davon, oder

(2) Durchführung einer Eliminierungsreaktion der Aminoschutzgruppe mit einer Verbindung der Fowmel:

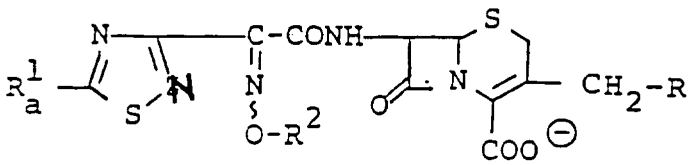

wobei R und $R^2$ jeweils wie oben definiert sind und $R_a{}^1$ eine geschützte Aminogruppe ist, oder eines Salzes davon zur Herstellung einer Verbindung der Formel:

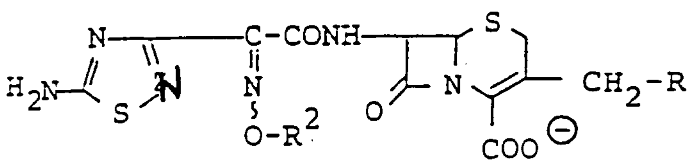

wobei R und $R^2$ jeweils wie oben definiert sind, oder eines Salzes davon, oder

(3) Durchführung einer Eliminierungsreaktion der Carboxyschutzgruppe mit einer Verbindung der Formel:

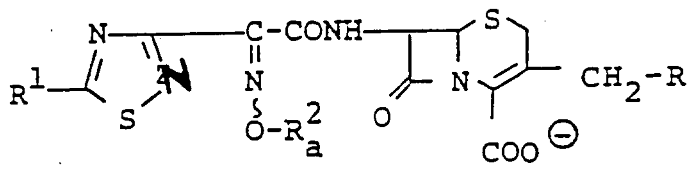

wobei R und $R^1$ jeweils wie oben definiert sind und $R^2{}_a$ geschütztes Carboxy($C_1$-$C_6$)alkyl ist, zur Herstellung einer Verbindung der Formel:

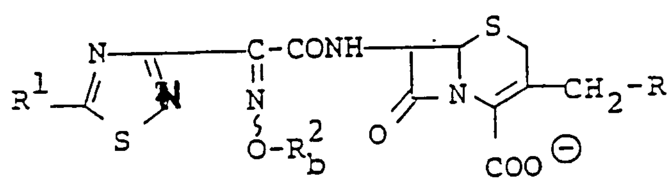

wobei R und $R^1$ jeweils wie oben definiert sind und $R^2{}_b$ Carboxy($C_1$-$C_6$)alkyl ist, oder eines Salzes davon, oder

(4) Umsetzung einer Verbindung der Formel:

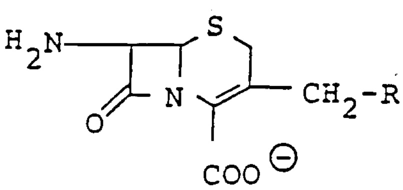

wobei R wie oben definiert ist,

oder ihres an der Aminogruppe reaktives Derivates oder eines Salzes davon mit einer Verbindung der Formel:

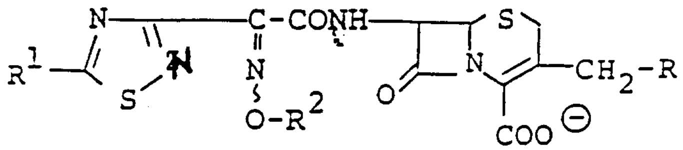

wobei $R^1$ und $R^2$ jeweils wie oben definiert sind, oder ihrem an der Carboxygruppe reaktiven Derivat oder einem Salz davon zur Herstellung einer Verbindung der Formel

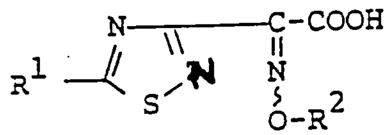

wobei R, $R^1$ und $R^2$ jeweils wie oben definiert sind, oder eines Salzes davon.

9. Eine Verbindung der Formel

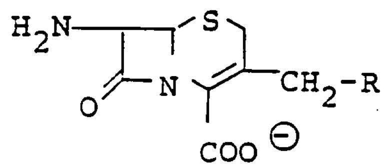

wobei R eine Gruppe der Formel ist:

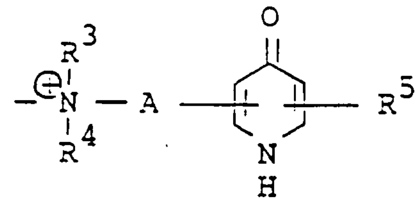

(wobei $R^3$ und $R^4$ jeweils ($C_1$-$C_6$) Alkyl sind, oder $R^3$ und $R^4$ unter Bildung von $C_3$-$C_6$ Alkylen aneinander gebunden sind, A ($C_1$-$C_6$) Alkylen ist, und
$R^5$ Hydroxy oder eine geschützte Hydroxygruppe ist),
oder ein Salz davon.

10. Ein Verfahren zur Herstellung einer Verbindung aus Anspruch 9 oder eines Salzes davon, welches umfaßt Durchführung einer Eliminierungsreaktion der Aminoschutzgruppe mit einer Verbindung der Formel

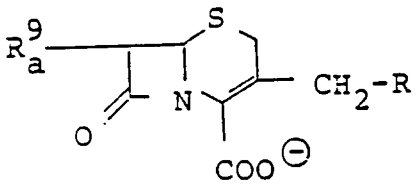

wobei R wie oben definiert ist und
$R_a^9$ eine geschützte Aminogruppe ist, oder einem Salzes davon.

11. Eine pharmazeutische Zusammensetzung, die als aktiven Bestandteil eine Verbindung aus Anspruch 1 oder ein pharmazeutisch verträgliches Salz davon im Gemisch mit pharmazeutisch verträglichen Trägern umfaßt.

12. Eine Verbindung aus Anspruch 1 oder pharmazeutisch verträgliche Salze davon für die Verwendung als Medikament.

13. Eine Verbindung aus Anspruch 1 oder pharmazeutisch verträgliche Salze davon für die Verwendung bei der Behandlung oder Vorbeugung von Infektionskrankheiten.

14. Verwendung einer Verbindung aus Anspruch 1 oder eines pharmazeutisch verträglichen Salzes davon für die Herstellung eines Medikamentes für die Behandlung oder Vorbeugung von Infektionskrankheiten.

**Patentansprüche für folgende Vertragsstaaten : ES, GR**

1. Verfahren zur Herstellung einer Verbindung der Formel

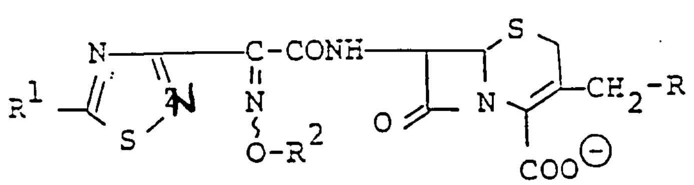

(I)

wobei $R^1$ Amino oder eine geschützte Aminogruppe ist,
$R^2$ $(C_1\text{-}C_6)$Alkyl, $(C_2\text{-}C_6)$Alkenyl, Carboxy$(C_1\text{-}C_6)$alkyl oder geschütztes Carboxy$(C_1\text{-}C_6)$alkyl ist und
R eine Gruppe der Formel ist:

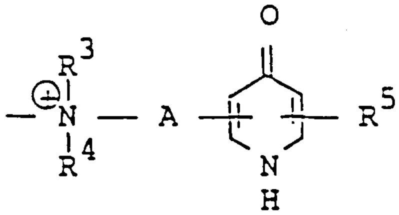

(wobei $R^3$ und $R^4$ jeweils$(C_1\text{-}C_6)$Alkyl ist, oder
$R^3$ und $R^4$ unter Bildung von$(C_3\text{-}C_6)$Alkylen aneinander gebunden sind, A $(C_1\text{-}C_6)$ Alkylen ist, und
$R^5$ Hydroxy oder eine geschützte Hydroxygruppe ist),
und ein pharmazeutisch verträgliches Salz davon, welches umfaßt:

(1) Umsetzung einer Verbindung der Formel:

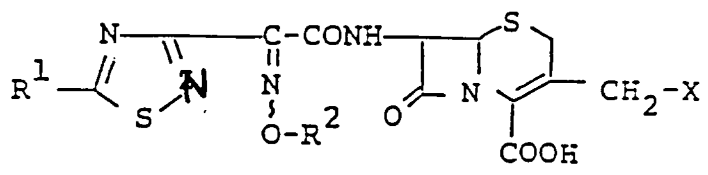

wobei $R^1$ und $R^2$ jeweils wie oben definiert sind und X ein Säurerest ist, oder eines Salzes davon mit einer Verbindung der Formel:

$R^a$

wobei $R^a$ eine Verbindung der Formel ist:

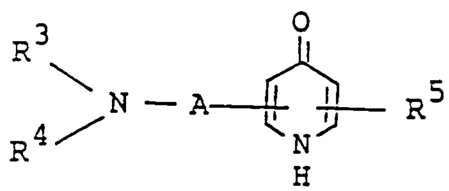

(wobei $R^3$, $R^4$, $R^5$ und A jeweils wie oben definiert sind), oder eines Salzes davon zur Herstellung einer Verbindung der Formel:

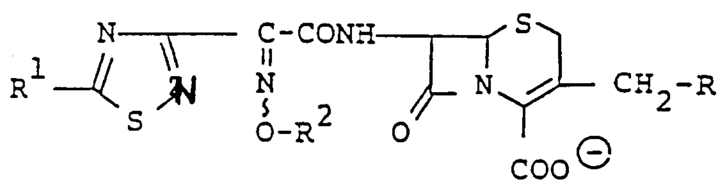

worin R, $R^1$ und $R^2$ jeweils wie oben definiert sind, oder eines Salzes davon, oder

(2) Durchführung einer Eliminierungsreaktion der Aminoschutzgruppe mit einer Verbindung der Formel:

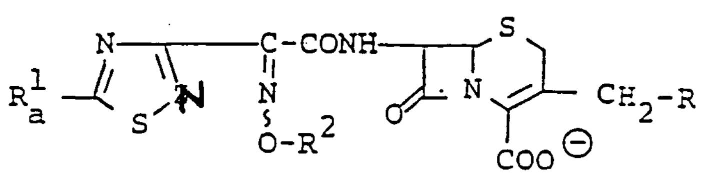

wobei R und $R^2$ jeweils wie oben definiert sind und $R_a^1$ eine geschützte Aminogruppe ist, oder eines Salzes davon zur Herstellung einer Verbindung der Formel:

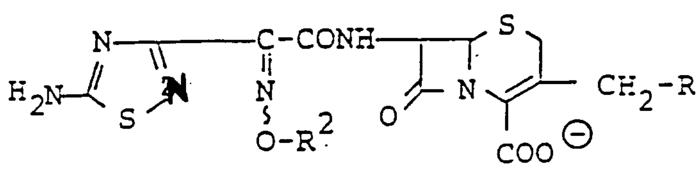

wobei R und $R^2$ jeweils wie oben definiert sind, oder eines Salzes davon, oder

(3) Durchführung einer Eliminierungsreaktion der Carboxyschutzgruppe mit einer Verbindung der Formel:

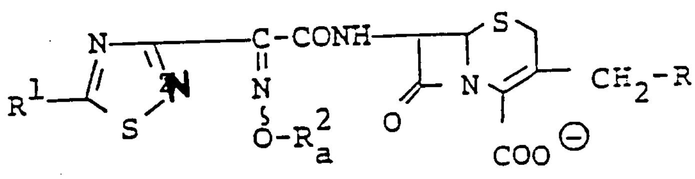

wobei R und $R^1$ jeweils wie oben definiert sind und $R^2_a$ geschütztes Carboxy($C_1$-$C_6$)alkyl ist, zur Herstellung einer Verbindung der Formel:

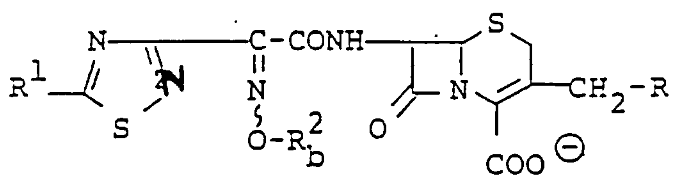

wobei R und $R^1$ jeweils wie oben definiert sind und $R^2_b$ Carboxy($C_1$-$C_6$)alkyl ist, oder eines Salzes davon, oder

(4) Umsetzung einer Verbindung der Formel:

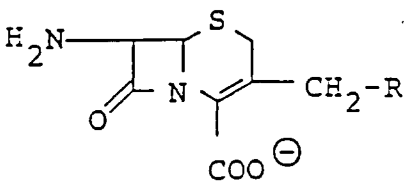

wobei R wie oben definiert ist, oder ihres an der Aminogruppe reaktives Derivates oder eines Salzes davon mit einer Verbindung der Formel:

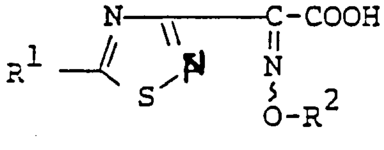

wobei $R^1$ und $R^2$ jeweils wie oben definiert sind, oder ihrem an der Carboxygruppe reaktiven Derivat oder einem Salz davon zur Herstellung einer Verbindung der Formel

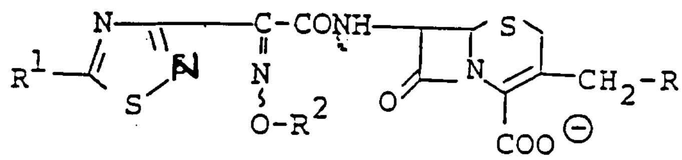

wobei R, $R^1$ und $R^2$ jeweils wie oben definiert sind, oder eines Salzes davon.

2. Verfahren aus Anspruch 1,
wobei $R^2$ Amino oder Acylamino ist und
$R^2$ ($C_1$-$C_6$) Alkyl, ($C_1$-$C_6$) Alkenyl, Carboxy($C_1$-$C_6$)alkyl oder verestertes Carboxy($C_1$-$C_6$)alkyl ist.

3. Verfahren aus Anspruch 2,
wobei $R^1$ Amino oder ($C_1$-$C_6$) Alkanoylamino ist.

4. Verfahren aus Anspruch 3,
wobei R eine Gruppe der Formel ist:

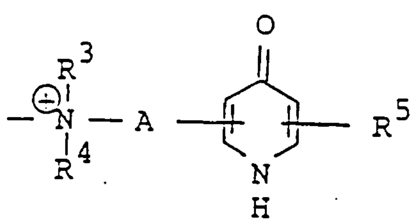

(wobei $R^3$ und $R^4$ jeweils ($C_1$-$C_6$) Alkyl ist,
$R^5$ Hydroxy ist und
A ($C_1$-$C_6$) Alkylen ist).

5. Verfahren aus Anspruch 4,
wobei $R^1$ Amino ist und
$R^2$ Carboxy($C_1$-$C_6$)alkyl ist.

6. Verfahren aus Anspruch 5 zur Herstellung von 7β-[2-(5-Amino-1,2,4-thiadiazol-3-yl)-2-(1-carboxy-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylat (syn-Isomer).

7. Verfahren aus Anspruch 5 zur Herstellung des Schwefelsäuresalzes von 7β-[2-(5-Amino-1,2,4-thiadiazol-3-yl)-2-(1-carboxyl-1-methylethoxyimino)acetamido]-3-[N,N-dimethyl-N-{(5-hydroxy-4-oxo-1,4-dihydropyridin-2-yl)methyl}ammonio]methyl-3-cephem-4-carboxylat (syn-Isomer).

8. Verfahren zur Herstellung einer Verbindung der Formel

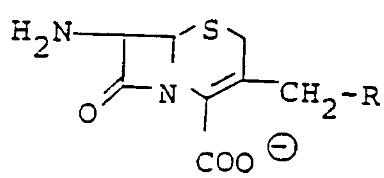

wobei R eine Gruppe der Formel ist:

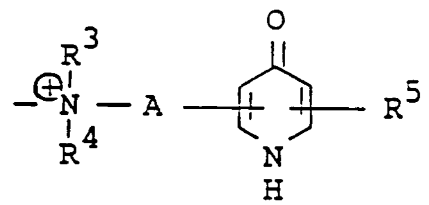

(wobei $R^3$ und $R^4$ jeweils ($C_1$-$C_6$) Alkyl ist, oder $R^3$ und $R^4$ unter Bildung von ($C_3$-$C_6$) Alkylen aneinander gebunden sind, A ($C_1$-$C_6$) Alkylen ist, und $R^5$ Hydroxy oder eine geschützte Hydroxygruppe ist), oder eines Salzes davon, welches umfaßt:
Durchführung einer Eliminierungsreaktion der Aminoschutzgruppe mit einer Verbindung der Formel:

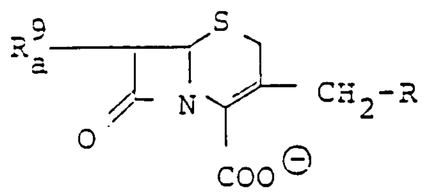

wobei R wie oben definiert ist und $R^9_a$ eine geschützte Aminogruppe ist, oder mit deren Salz.

9. Verfahren zur Herstellung einer pharmazeutischen Zusammensetzung, das dadurch charakterisiert ist, daß eine Verbindung der Formel I oder ein nicht-toxisches Salz davon, hergestellt nach einem in Anspruch 1 beanspruchten Verfahren durch Mischen oder Verarbeiten dieser Verbindung mit einem pharmazeutisch verträglichen Verdünner oder Träger in pharmazeutisch verträgliche Form gebracht wird.

**Revendications**

**Revendications pour les Etats contractants suivants : AT, BE, CH, DE, FR, GB, IT, LI, LU, NL, SE**

1. Composé de la formule :

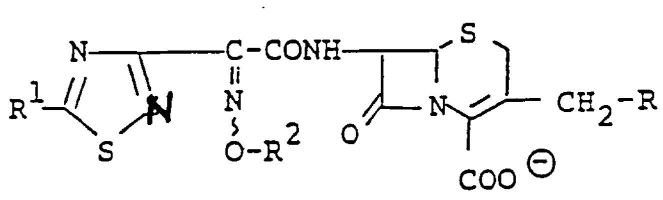

dans laquelle

$R^1$ est un groupe amino ou amino protégé,
$R^2$ est un groupe alkyle en $C_1$-$C_6$, alcényle en $C_2$-$C_6$, carboxy(alkyle en $C_1$-$C_6$) ou carboxy(alkyle en $C_1$-$C_6$)protégé, et

R est un groupe de la formule :

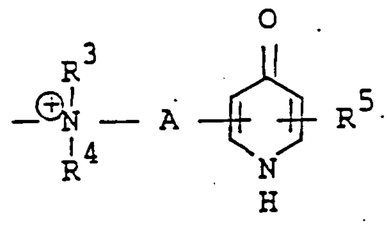

(dans laquelle

| | |
|---|---|
| $R^3$ et $R^4$ | sont chacun un groupe alkyle en $C_1$-$C_6$, ou |
| $R^3$ et $R^4$ | sont liés pour former un groupe alkylène en $C_3$-$C_6$, |
| A | est un groupe alkylène en $C_1$-$C_6$, et |
| $R^5$ | est un groupe hydroxy ou hydroxy protégé), |

et un de ses sels pharmaceutiquement acceptables.

2. Composé selon la revendication 1, dans laquelle

$R^1$ est un groupe amino ou acylamino, et
$R^2$ est un groupe alkyle en $C_1$-$C_6$, alcényle en $C_1$-$C_6$, carboxy(alkyle en $C_1$-$C_6$) ou carboxy(alkyle en $C_1$-$C_6$) estérifié.

3. Composé selon la revendication 2, dans lequel $R^1$ est un groupe amino ou alcanoylamino en $C_1$-$C_6$.

4. Composé selon la revendication 3, dans lequel R est un groupe de la formule :

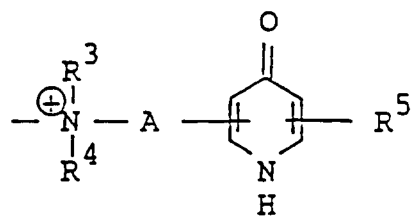

(dans laquelle

| | |
|---|---|
| $R^3$ et $R^4$ | sont chacun un groupe alkyle en $C_1$-$C_6$, |
| $R^5$ | est un groupe hydroxy et |
| A | est un groupe alkylène en $C_1$-$C_6$). |

5. Composé selon la revendication 4, dans lequel

$R^1$ est un groupe amino et
$R^2$ est un groupe carboxy(alkyle en $C_1$-$C_6$).

6. Composé selon la revendication 5, qui est le 7β-[2-(5-amino-1,2,4-thiadiazol-3-yl)-2-(1-carboxy-1-méthyléthoxyimino)acétamido]-3-[N,N-diméthyl-N-{(5-hydroxy-4-oxo-1,4-dihydropyridin-2-yl)méthyl}ammonio]méthyl-3-céphem-4-carboxylate (isomère syn).

7. Composé selon la revendication 5, qui est le sel d'acide sulfurique du 7β-[2-(5-amino-1,2,4-thiadiazol-3-yl)-2-(1-carboxy-1-méthyléthoxyimino)acétamido]-3-[N,N-diméthyl-N-{(5-hydroxy-4-oxo-1,4-dihydropyridin-2-yl)méthyl}ammonio]méthyl-3-céphem-4-carboxylate (isomère syn).

8. Procédé pour préparer un composé de la revendication 1 ou un de ses sels, qui comprend :

(1) le fait de faire réagir un composé de la formule :

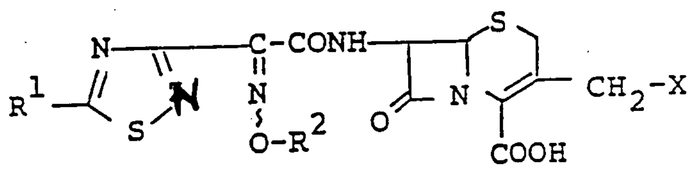

dans laquelle $R^1$ et $R^2$ sont chacun tels que définis ci-dessus et

X est un résidu acide,

ou un de ses sels, avec un composé de la formule :

$R^a$

où $R^a$ est un composé de la formule :

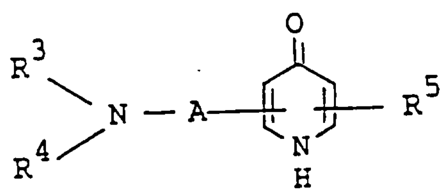

(dans laquelle $R^3$, $R^4$, $R^5$ et A sont chacun tels que définis ci-dessus, ou un de ses sels, pour donner un composé de la formule :

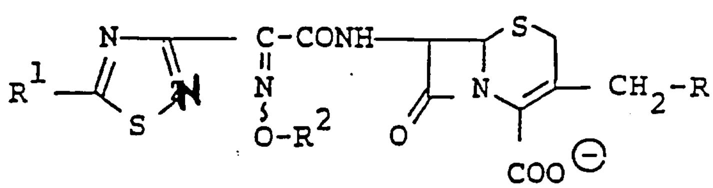

dans laquelle R, $R^1$ et $R^2$ sont chacun tels que définis ci-dessus, ou un de ses sels, ou
(2) le fait de soumettre un composé de la formule :

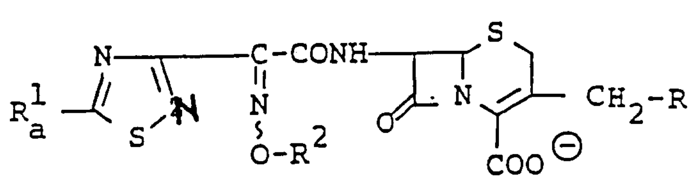

dans laquelle R et $R^2$ sont chacun tels que définis ci-dessus et

$R_a^1$ est un groupe amino protégé,

ou un de ses sels, à une réaction d'élimination du groupe protecteur du groupe amino pour donner un composé de la formule :

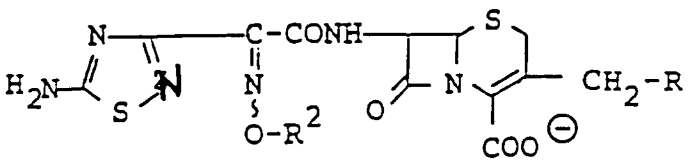

dans laquelle R et $R^2$ sont chacun tels que définis ci-dessus,
ou un de ses sels, ou
(3) le fait de soumettre un composé de la formule :

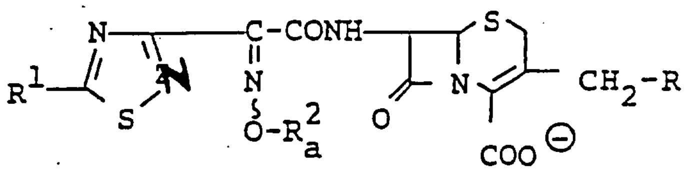

dans laquelle R et $R^1$ sont chacun tels que définis ci-dessus et

$R_a^2$ est un groupe carboxy(alkyle en $C_1$-$C_6$) protégé,

ou un de ses sels, à une réaction d'élimination du groupe protecteur du groupe carboxy pour donner un composé de la formule :

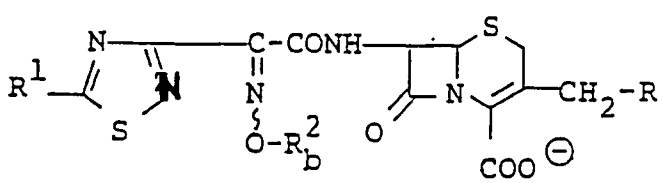

dans laquelle R et $R^1$ sont chacun tels que définis ci-dessus, et

$R_b^2$ est un groupe carboxy(alkyle en $C_1$-$C_6$),

ou un de ses sels, ou
(4) le fait de faire réagir un composé de la formule :

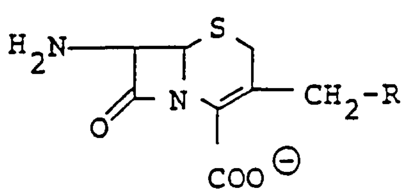

dans laquelle R est tel que défini ci-dessus,
ou son dérivé réactif au groupe amino,

ou un de ses sels, avec un composé de la formule :

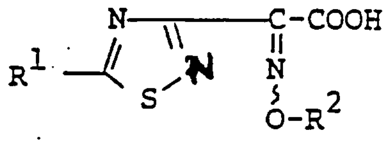

dans laquelle $R^1$ et $R^2$ sont chacun tels que définis ci-dessus,
ou son dérivé réactif au groupe carboxy,
ou un de ses sels, pour donner un composé de la formule :

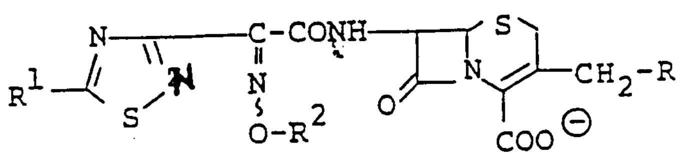

dans laquelle R, $R^1$ et $R^2$ sont chacun tels que définis ci-dessus,
ou un de ses sels.

**9.** Composé de la formule :

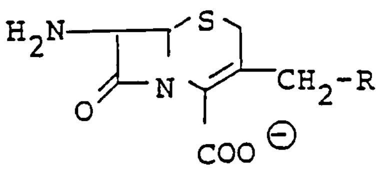

dans laquelle R est un groupe de la formule :

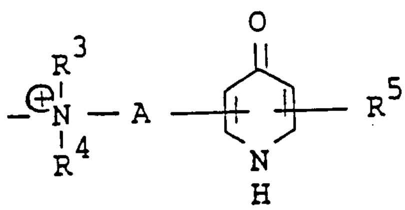

(dans laquelle

| | |
|---|---|
| $R^3$ et $R^4$ | sont chacun un groupe alkyle en $C_1$-$C_6$, ou |
| $R^3$ et $R^4$ | sont liés pour former un groupe alkylène en $C_3$-$C_6$, |
| A | est un groupe alkylène en $C_1$-$C_6$, et |
| $R^5$ | est un groupe hydroxy ou hydroxy protégé), |

ou un de ses sels.

**10.** Procédé pour préparer un composé selon la revendication 9
ou un de ses sels,
qui consiste à soumettre un composé de la formule :

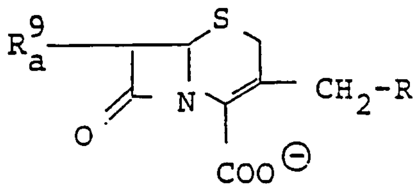

dans laquelle

R est tel que défini ci-dessus, et
$R^9_a$ est un groupe amino protégé,

ou un de ses sels, à une réaction d'élimination du groupe protecteur du groupe amino.

11. Composition pharmaceutique qui comprend, comme ingrédient actif, un composé de la revendication 1 ou un de ses sels pharmaceutiquement acceptables en mélange avec des supports pharmaceutiquement acceptables.

12. Composé selon la revendication 1 ou ses sels pharmaceutiquement acceptables pour emploi comme médicament.

13. Composé selon la revendication 1, ou ses sels pharmaceutiquement acceptables pour emploi dans le traitement ou la prévention des maladies infectieuses.

14. Utilisation d'un composé de la revendication 1 ou d'un de ses sels pharmaceutique pour la fabrication d'un médicament destiné au traitement ou à la prévention des maladies infectieuses.

**Revendications pour les Etats contractants suivants : ES, GR**

1. Procédé pour la préparation d'un composé de la formule :

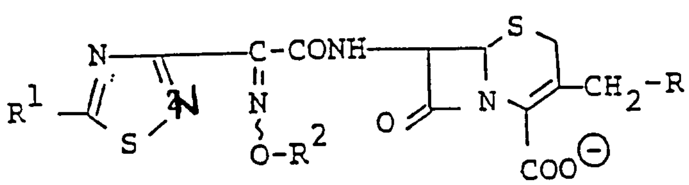

(I)

dans laquelle

$R^1$ est un groupe amino ou amino protégé,
$R^2$ est un groupe alkyle en $C_1$-$C_6$, alcényle en $C_2$-$C_6$, carboxy(alkyle en $C_1$-$C_6$) ou carboxy(alkyle en $C_1$-$C_6$)protégé, et
R est un groupe de la formule :

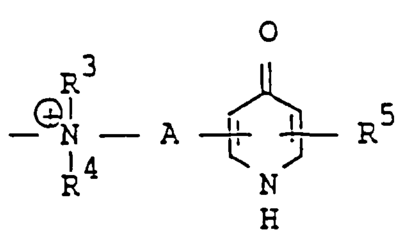

(dans laquelle

| | |
|---|---|
| $R^3$ et $R^4$ | sont chacun un groupe alkyle en $C_1$-$C_6$, ou |
| $R^3$ et $R^4$ | sont liés pour former un groupe alkylène en $C_3$-$C_6$, |
| A | est un groupe alkylène en $C_1$-$C_6$, et |
| $R^5$ | est un groupe hydroxy ou hydroxy protégé), |

ou un de ses sels, qui comprend :

(1) le fait de faire réagir un composé de la formule :

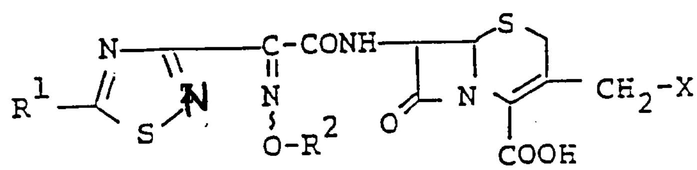

dans laquelle $R^1$ et $R^2$ sont chacun tels que définis ci-dessus et

X est un résidu acide,

ou un de ses sels, avec un composé de la formule :

$R^a$

où $R^a$ est un composé de la formule :

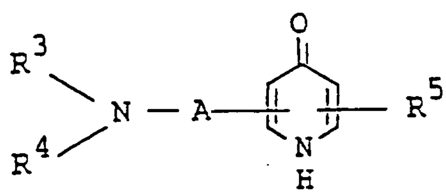

(dans laquelle $R^3$, $R^4$, $R^5$ et A sont chacun tels que définis ci-dessus, ou un de ses sels, pour donner un composé de la formule :

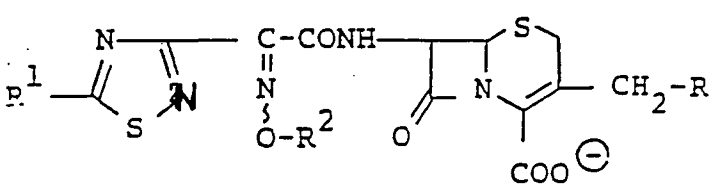

dans laquelle R, $R^1$ et $R^2$ sont chacun tels que définis ci-dessus, ou un de ses sels, ou

(2) le fait de soumettre un composé de la formule :

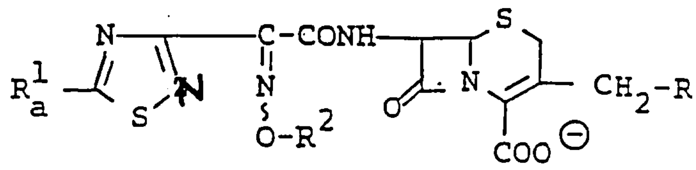

dans laquelle R et $R^2$ sont chacun tels que définis ci-dessus et

$R^1_a$ est un groupe amino protégé,

ou un de ses sels, à une réaction d'élimination du groupe protecteur du groupe amino pour donner un composé de la formule :

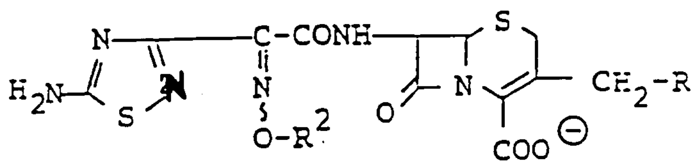

dans laquelle R et $R^2$ sont chacun tels que définis ci-dessus,
ou un de ses sels, ou
(3) le fait de soumettre un composé de la formule :

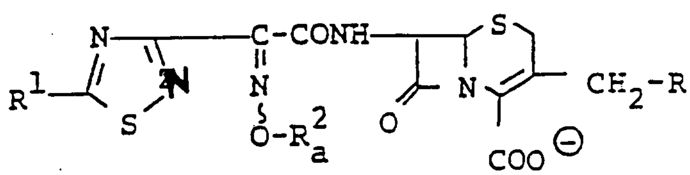

dans laquelle R et $R^1$ sont chacun tels que définis ci-dessus et

$R^2_a$ est un groupe carboxy(alkyle en $C_1$-$C_6$) protégé,

ou un de ses sels, à une réaction d'élimination du groupe protecteur du groupe carboxy pour donner un composé de la formule :

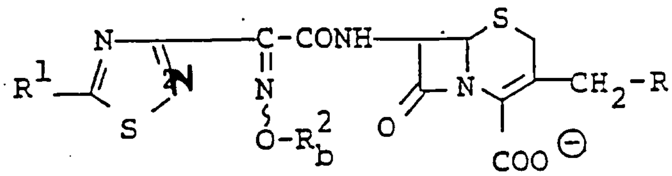

dans laquelle R et $R^1$ sont chacun tels que définis ci-dessus, et

$R^2_b$ est un groupe carboxy(alkyle en $C_1$-$C_6$),

ou un de ses sels, ou

(4) le fait de faire réagir un composé de la formule :

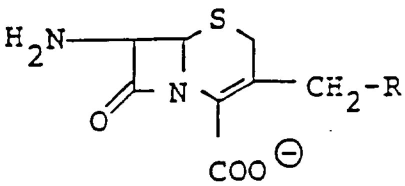

dans laquelle R est tel que défini ci-dessus,
ou son dérivé réactif au groupe amino,
ou un de ses sels, avec un composé de la formule :

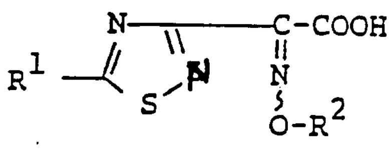

dans laquelle $R^1$ et $R^2$ sont chacun tels que définis ci-dessus,
ou son dérivé réactif au groupe carboxy,
ou un de ses sels, pour donner un composé de la formule :

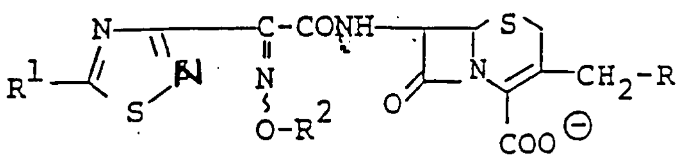

dans laquelle R, $R^1$ et $R^2$ sont chacun tels que définis ci-dessus,

ou un de ses sels.

2. Procédé selon la revendication 1, dans lequel

$R^1$ est un groupe amino ou acylamino, et
$R^2$ est un groupe alkyle en $C_1$-$C_6$, alcényle en $C_1$-$C_6$, carboxy(alkyle en $C_1$-$C_6$) ou carboxy(alkyle en $C_1$-$C_6$) estérifié.

3. Procédé selon la revendication 2, dans lequel

$R^1$ est un groupe amino ou alcanoylamino en $C_1$-$C_6$.

4. Procédé selon la revendication 3, dans lequel R est un groupe de la formule :

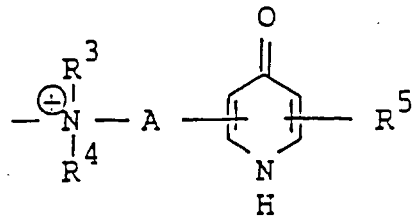

(dans laquelle

$R^3$ et $R^4$ sont chacun un groupe alkyle en $C_1$-$C_6$,
$R^5$ est un groupe hydroxy et
A est un groupe alkylène en $C_1$-$C_6$).

5. Procédé selon la revendication 4, dans lequel

$R^1$ est un groupe amino et
$R^2$ est un groupe carboxy(alkyle en $C_1$-$C_6$).

6. Procédé selon la revendication 5 pour la préparation du 7β-[2-(5-amino-1,2,4-thiadiazol-3-yl)-2-(1-carboxy-1-méthyléthoxyimino)acétamido]-3-[N,N-diméthyl-N-{(5-hydroxy-4-oxo-1,4-dihydropyridin-2-yl)méthyl}ammonio]méthyl-3-céphem-4-carboxylate (isomère syn).

7. Procédé selon la revendication 5 pour la préparation du sel d'acide sulfurique du 7β-[2-(5-amino-1,2,4-thiadiazol-3-yl)-2-(1-carboxy-1-méthyléthoxyimino)acétamido]-3-[N,N-diméthyl-N-{(5-hydroxy-4-oxo-1,4-dihydropyridin-2-yl)méthyl}ammonio]méthyl-3-céphem-4-carboxylate (isomère syn).

8. Procédé pour la préparation d'un composé de la formule :

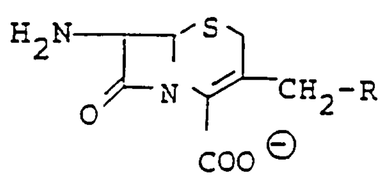

dans laquelle R est un groupe de la formule :

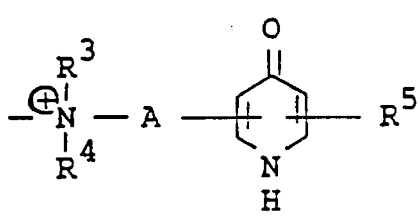

(dans laquelle

$R^3$ et $R^4$ sont chacun un groupe alkyle en $C_1$-$C_6$, ou
$R^3$ et $R^4$ sont liés pour former un groupe alkylène en $C_3$-$C_6$,
A est un groupe alkylène en $C_1$-$C_6$, et
$R^5$ est un groupe hydroxy ou hydroxy protégé),

ou un de ses sels,
qui comprend le fait de soumettre un composé de la formule :

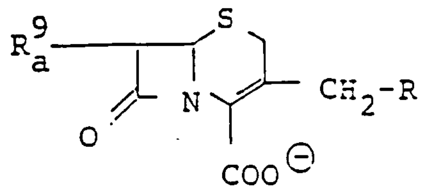

dans laquelle

R est tel que défini ci-dessus, et
$R_a^9$ est un groupe amino protégé,

ou un de ses sels, à une réaction d'élimination du groupe protecteur du groupe amino.

**9.** Procédé pour la préparation d'une composition pharmaceutique qui est caractérisé en ce qu'on met un composé de la formule I, ou un de ses sels non-toxiques, obtenu par le procédé revendiqué en revendication 1, sous forme pharmaceutiquement acceptable par mélange ou présentation dudit composé avec un diluant ou un support pharmaceutiquement acceptable.